# EUROPÄISCHE PATENTANMELDUNG

(11) **EP 2 774 920 A1**
(43) Veröffentlichungstag der Anmeldung: **10.09.2014**
(21) Anmeldenummer: 14001134.7
(22) Anmeldetag: 01.03.2011
(51) Int. Cl.: C07D 237/32, A61K 31/502, A61P 37/02

(54) **KRISTALLINE FORMEN VON 5-AMINO-2,3-DIHYDROPHTHALAZIN-1,4-DION NATRIUMSALZ UND IHRE MEDIZINISCHE VERWENDUNG**

(30) Priorität: 01.03.2010 EP 10002067; 25.11.2010 EP 10075744
(62) Teilanmeldung aus: 11710423.2
(71) Anmelder: MetrioPharm AG, 8002 Zürich (CH)
(72) Erfinder: Breu, Josef, 95448 Bayreuth (DE); Brysch, Wolfgang, 13505 Berlin (DE); Kaiser, Astrid, 12305 Berlin (DE); Ludescher, Beate, 13439 Berlin (DE); Maass, Gerrit, 30657 Hannover (DE); Martin, Thomas, 95100 Selb (DE); Milius, Wolfgang, 95447 Bayreuth (DE); Niedermaier, Michael, 12359 Berlin (DE)
(74) Vertreter: Seuss, Thomas

(57) **Zusammenfassung**

Die Erfindung betrifft die Bereitstellung zweier neuer kristalliner Formen I und II zu 5-Amino-2,3-dihydrophthalazin-1,4-dion Natriumsalz. Es wurde überraschenderweise gefunden, dass Form I und Form II unterschiedliche immunologische Wirkungen aufweisen. Diese vorteilhafte Eigenschaft ist nützlich für immunspezifische Anwendungen. Des Weiteren verfügen beide Formen über vorteilhafte physikochemische Eigenschaften, welche nützlich sind bei der Herstellung, Weiterverarbeitung und / oder Anwendung einer pharmazeutischen Zubereitung aus Form I oder Form II oder einer Mischung aus beiden.

## Beschreibung

### TECHNISCHES GEBIET DER ERFINDUNG:

Die vorliegende Erfindung betrifft die Bereitstellung von mindestens zwei neuen kristallinen Formen zu 5-Amino-2,3-dihydrophthalazin-1,4-dion Natriumsalz mit vorteilhaften Eigenschaften, diese enthaltende pharmazeutische Zubereitungen, sowie Verfahren zu deren Herstellung.

Die Erfindung betrifft insbesondere die Bereitstellung von 2 neuen kristallinen Formen zu 5-Amino-2,3-dihydrophthalazin-1,4-dion Natriumsalz mit immunstimulierenden und immunsupprimierenden Eigenschaften zu medizinischen Zwecken.

### HINTERGRUND DER ERFINDUNG:

Seit längerem sind aus dem Stand der Technik chemische Verbindungen mit immunmodulatorischer Wirkung bekannt. Zu diesen Verbindungen zählt mit 5-Amino-2,3-dihydrophthalazin-1,4-dion Natriumsalz auch dasjenige der vorliegenden Erfindung, welches zum Beispiel aus der EP 1 203 587 A bekannt ist und die folgende Grundstruktur besitzt (Na+ nicht gezeigt):

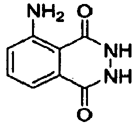

Diese oben gezeigte Grundstruktur wird auch als Luminol bezeichnet. Aus dem Stand der Technik ist bekannt, dass 5-Amino-2,3-dihydrophthalazin-1,4-dion Alkalisalze als Feststoffe in verschiedenen Hydratformen kristallisieren. Im Stand der Technik sind insbesondere das Dihydrat des Natriumsalzes (RU 2113222 C1) und ein Trihydrat des Kaliumsalzes beschrieben sowie Mischformen hierzu (RU2211036 C2).

Es ist bekannt, dass kristalline Formen einer Substanz in ihren physikalischen Eigenschaften wie Löslichkeit, Auflöserate, Stabilität usw. divergieren können (Haleblian and McCrone (1969): Journal of Pharmaceutical Sciences, 58:911-929.).

Solche Eigenschaften können die pharmazeutische Verarbeitung des Wirkstoffs ebenso beeinflussen wie seine biologische Verfügbarkeit und mithin die biologische Wirksamkeit (vgl. Griesser (2006) in: Polymorphisms in the Pharmaceutical Industry. Hilfiker (Ed.) 211-234)

Für die Arzneimittelherstellung ist es wichtig, dass der Ausgangsstoff stabil ist, nicht wasserziehend und in seinem Feststoffverhalten über den gesamten Herstellungsprozess kontrollierbar. Zudem ist die chemische Stabilität und Festphasenstabilität mit langer Lagerfähigkeit eines Wirkstoffs von herausragender Bedeutung (vgl. Miller et al. (2006) in: Polymorphisms in the Pharmaceutical Industry. Hilfiker (Ed.) 385-403). Dabei ist es wünschenswert, dass auch über eine längere Lagerperiode die physikalischen Eigenschaften des Wirkstoffs erhalten bleiben. Dies betrifft z.B. die Hygroskopizität, Löslichkeit oder initiale Auflösungsrate des Wirkstoffs.

Die US 6,489,326 B1 beschreibt ein Verfahren zur Herstellung eines 5-Amino-2,3-dihydrophthalazin-1,4-dion Natriumsalzes zur Verwendung für medizinische Zwecke, welches eine Dihydratform zum Ergebnis hat. Nachteil dieses Verfahrens ist der Einsatz eines Schwermetallkatalysators, der Rückstände im Produkt hinterlassen kann. Rückstandsbehafte Produkte besitzen allergenes Potential und werden bei einer pharmazeutischen Verwendung von der EMEA allgemein kritisch betrachtet (vgl. Richtlinie EMEA/CHMP/SWP/4446/2000).

Von großer Bedeutung für die pharmazeutische Verarbeitung und die medizinische Verwendung sind Herstellungsverfahren, welche die Herstellung der gewünschten kristallinen Formen verlässlich und reproduzierbar ermöglichen. Bei der Herstellung kristalliner Formen ist zu bedenken, dass bereits geringe Abweichungen in den Prozessparametern Änderungen in der Kristallstruktur des Produkts verursachen und damit im Ergebnis zu anderen kristallinen Formen bzw. Mischformen führen können. Dadurch veränderte Eigenschaften - zum Beispiel eine veränderte biologische Wirksamkeit durch eine andere Löslichkeit - können zum Ausfall ganzer Chargen führen, mitunter ist es gar nicht mehr möglich, die gewünschte Form herzustellen (vgl. Ulrich und Jones (2005): Nachrichten aus der Chemie 53:19-23). Neben der Reinheit des Wirkstoffs und den sich daraus ergebenden möglichen Änderungen der Wirksamkeit können so auch weitere wichtige Eigenschaften für die pharmazeutische Verarbeitung nachteilhaft beeinflusst werden, z.B. die Eignung zur Tablettenverpressung durch eine Beeinträchtigung der Rieselfähigkeit oder Fließgeschwindigkeit der kristallinen Form. 5-Amino-2,3-dihydrophthalazin-1,4-dion Alkalisalze gehören zur Gruppe der Aminophthalhydrazide und werden im Stand der Technik beschrieben als Immunmodulatoren mit besonderen anti-inflammatorischen, anti-oxidativen und anti-toxischen Eigenschaften (vgl. US 6,489,326 B1; EP 0617024, US 5,512,573, US 5,543,410 A, US 7,326,690 B2).

Immunmodulatorische Substanzen werden üblicherweise ihrer Wirkung entsprechend in Immunsuppressiva und Immunstimulanzien unterteilt (vgl. Rote Liste Service GmbH (2011): www.rote-liste.de).

Die entsprechenden Präparate mit ausschließlich immunsupprimierender oder ausschließlich immunstimulierender Wirkung, wie zum Beispiel immunsupprimierende TNF-alpha-Blocker oder immunstimulierende Interferon-beta-Präparate, rufen häufig gerade aufgrund ihres sehr spezifischen Wirkmechanismus erhebliche unerwünschte Nebenwirkungen im Organismus hervor. Einige bekannte immunsupprimierende Substanzen, wie beispielsweise der TNF-alpha-Blocker Adalimumab, hemmen gezielt bestimmte Entzündungsmediatoren. Derartige Therapien sind bekanntermaßen mit gravierenden Nebenwirkungen verbunden (vgl. Descotes (2008): Expert Opin. Drug Metab. Toxicol., 4:12:1537-1549), da die Blockade einzelner Entzündungsmediatoren einen schweren Eingriff in das komplexe Immunsystem darstellt. Infolge dessen ist der Organismus nicht mehr in der Lage seine Funktion zu erfüllen, d.h. selbständig und physiologisch angemessen auf exogene oder endogene Entzündungsreize, wie z.B. bakterielle Infektionen zu reagieren. So ist zum Beispiel der Einsatz von TNF-alpha-Blockern im Falle schwerwiegender Infektionen kontraindiziert, dies gilt insbesondere für Sepsis und Tuberkulose. So wird vor der Gabe einer entsprechenden Medikation, wie z.B. bei der Behandlung Rheumatoider Arthritis, ein TBC-Screening dringend empfohlen (vgl. Diel et al. (2009): Z Rheumatol 5:411-416). Außerdem konnte durch Hoffmann (2005: Intensivmed 42:371-377) anschaulich gezeigt werden, dass sich TNF-alpha-Blocker nicht für die klinische Anwendung bei septischen Zuständen eignen, sondern im Gegenteil sogar zu einer Erhöhung der Sterblichkeit führen können.

Die besonderen immunmodulatorischen Eigenschaften der 5-Amino-2,3-dihydrophthalazin-1,4-dion Alkalisalze dagegen erweisen sich als ausgesprochen nützlich bei der Bekämpfung von sogenannten Zytokinstürmen (vgl. Suntharalingam et al. (2006): N Engl J Med 355;1018-28) infolge überschießender Immunreaktionen. Denn im Unterschied zu sogenannten Zytokinblockern sind diese Salze weitestgehend nebenwirkungsfrei, da es nicht zu einer Hemmung einzelner Zytokine kommt, sondern diese auf ein physiologisches Niveau reguliert werden und somit weiterhin eine adäquate Reaktion des Organismus auf infektiöse Keime gewährleistet ist. Allerdings weist der Stand der Technik einzelnen kristallinen Formen von Alkalisalzen zu 5-Amino-2,3-dihydrophthalazin-1,4-dion keine unterschiedlichen immunspezifischen Wirkungen zu. Vor allem lassen sich dem Stand der Technik keine Aussagen darüber entnehmen, ob einzelne kristalline Formen zielgerichtet und indikationsgemäß, d.h. nach ihrer zugrunde liegenden, spezifischen immunmodulatorischen Hauptwirkung, bevorzugt als Immunsuppressiva oder bevorzugt als Immunstimulanzien eingesetzt werden können.

Eine besondere medizinische Verwendung des 5-Amino-2,3-dihydrophthalazin-1,4-dion Natriumsalzes beschreibt die US2003/0195183 A1 mit der "Korrektur des Immunsystems" durch die Verwendung unterschiedlicher Dosierungen (0,2 µg bis zu 1.000 mg) von 5-Amino-2,3-dihydrophthalazin-1,4-dion Alkalisalzen in verschiedenen Versuchsansätzen. Effektive Dosierungen innerhalb dieses Bereiches variierten je nach untersuchter Krankheit und individuellen Parametern wie z.B. Spezies, Alter, Geschlecht, und Gewicht. So führt zum Beispiel die Anwendung verschiedener Dosierungen zwischen 2 und 200 µg von 5-Amino-2,3-dihydrophthalazin-1,4-dion Natriumsalz zu unterschiedlichen Auswirkungen auf die durch subkutane Erythrozyteninjektion ausgelöste zelluläre Immunreaktion bei Mäusen (verzögerte Hypersensitivitätsreaktion - DHR). Je niedriger die verwendete Dosis, desto höher ist der DHR-Index. In dem sensitiveren der zwei verwendeten Mäusestämme führt die höchste verwendete Dosierung zu einer Hemmung der DHR. Dieses und weitere in vivo, in vitro und auch klinische Beispiele in der US2003/0195183 A1 deuten daraufhin, dass niedrige Dosierungen von 5-Amino-2,3-dihydrophthalazin-1,4-dion Alkalisalzen vorwiegend immunstimulierend wirken, während höhere Dosierungen von 5-Amino-2,3-dihydrophthalazin-1,4-dion Alkalisalzen vorwiegend immunsupprimierend wirken. Da der Übergang von immunstimulierender Dosierung zu immunsupprimierender Dosierung bei unterschiedlichen Mäusestämmen unterschiedlich hoch liegt, sind auch bei der Behandlung unterschiedlicher Spezies und Individuen in der Human- und Veterinärmedizin populationsgenetische Effekte zu erwarten, wodurch sich besondere Gefahren dieser dosisabhängigen Anwendung ergeben.

Von Vorteil für die klinische Praxis, insbesondere bei der Bekämpfung schwerwiegender Krankheiten mit angeborener oder erworbener Immundefizienz oder mit überschießenden Immunreaktionen, wäre folglich eine Methode zur gezielten Steuerung der Immunmodulation durch die einfache Bereitstellung unterschiedlicher Polymorphen einer Substanz.

Die Eigenschaft eines vorzugsweise stimulierend wirkenden Immunmodulators ist wünschenswert beispielsweise zur therapeutischen Behandlung von Patienten, die über eine geschwächte Immunabwehr verfügen, wie etwa in Folge einer HIV-Infektion oder infolge einer chemotherapeutischen Behandlung.

Die Eigenschaft eines vorzugsweise supprimierend wirkenden Immunmodulators ist wünschenswert, beispielsweise zur Minimierung von Entzündungsprozessen, etwa im Rahmen eines chirurgischen Eingriffs, bei Autoimmunerkrankungen und bei Allergien.

In der klinischen Praxis werden für manche Indikationen, insbesondere schubförmig verlaufende entzündliche Erkrankungen, häufig sowohl Immunstimulanzien als auch Immunsuppressiva gleichzeitig oder alternierend therapeutisch eingesetzt (vgl. DMSG 2006: Aktuelle Therapieempfehlungen September 2006; Rote Liste Service GmbH (2011): www.rote-liste.de). Dies führt zu einer erhöhten Gefahr von Wechselwirkungen. Chemisch ähnliche Substanzen einer Substanzklasse, die imstande sind, entgegengesetzte Wirkungen zu entfalten, würden folglich einen Vorteil für die klinische Praxis bieten, weil zu erwarten ist, dass sie bei gleichzeitiger oder zeitversetzter Gabe im Organismus weniger pharmakologisch bedingte Wechselwirkungen hervorrufen, als zu den gleichen Zwecken eingesetzte Wirkstoffe unterschiedlicher Substanzklassen.

Ein Nachteil des Standes der Technik besteht ferner darin, dass eine dosisabhängige Applikation eines Immunmodulators eine erhöhte Aufmerksamkeit des applizierenden medizinischen Personals oder des Patienten selbst erfordert und damit das Risiko von Anwendungsfehlern erhöht.

Wünschenswert ist daher eine nicht nur dosisabhängige immunspezifische Anwendung eines 5-Amino-2,3-dihydrophthalazin-1,4-dion Alkalisalzes für eine praktikable und risikoärmere therapeutische Anwendung, welche zugleich die medizinischen Vorteile dieser Substanzklasse ausschöpft.

Es bleibt festzuhalten, dass Eingriffe in das komplexe Regelwerk des Immunsystems schwerwiegende Folgen für den betroffenen Organismus bedeuten können. Umso wichtiger für die medizinische Anwendung sind daher Immunmodulatoren mit möglichst geringen Nebenwirkungen und immunologisch möglichst definierter Wirkung, welche eine gezielte Prophylaxe oder Therapie immunologisch bedingter Krankheiten ermöglichen. Solche Immunmodulatoren wären für die Medizin von großer Bedeutung.

### ZUSAMMENFASSUNG DER ERFINDUNG

Die vorliegende Erfindung wurde vor dem Hintergrund des vorstehend beschriebenen Standes der Technik gemacht, wobei es Aufgabe der vorliegenden Erfindung war, neue Formen zu 5-Amino-2,3-dihydrophthalazin-1,4-dion Natriumsalz mit spezifischen, voneinander abgrenzbaren immunologischen Wirkungen bereitzustellen, die solcherart gezielt für vorwiegend immunsupprimierende oder vorwiegend immunstimulierende Zwecke eingesetzt werden können. Als besonders wünschenswerte Eigenschaft sollen diese neuen Formen dosisunabhängig jeweils überwiegend immunstimulierende oder überwiegend immunsupprimierende Wirkungen zeigen. Darüber hinaus sollen die bereitgestellten Formen physikalisch-chemische Eigenschaften aufweisen, welche für daraus einzeln oder in Kombination hergestellte, gelagerte und/oder angewendete Arzneimittel vorteilhaft sind.

Die Aufgabe wurde durch die Bereitstellung von zwei neuen Anhydraten des 5-Amino-2,3-dihydrophthalazin-1,4-dion Natriumsalzes (Anhydratformen I und II) gelöst, welche sich überraschenderweise anhand experimenteller Daten in physikochemischen und voneinander abgrenzbaren immunmodulatorischen Eigenschaften vom Stand der Technik nachweislich abheben, insbesondere also vom bekannten 5-Amino-2,3-dihydrophthalazin-1,4-dion Natriumsalz. Während die erfindungsgemäße Anhydratform I (→kristalline Form I) über immunmodulatorische, insbesondere überwiegend immunstimulierende Eigenschaften verfügt, besitzt die neue erfindungsgemäße Anhydratform II (→ kristalline Form II) immunmodulatorische, insbesondere überwiegend immunsupprimierende Eigenschaften.

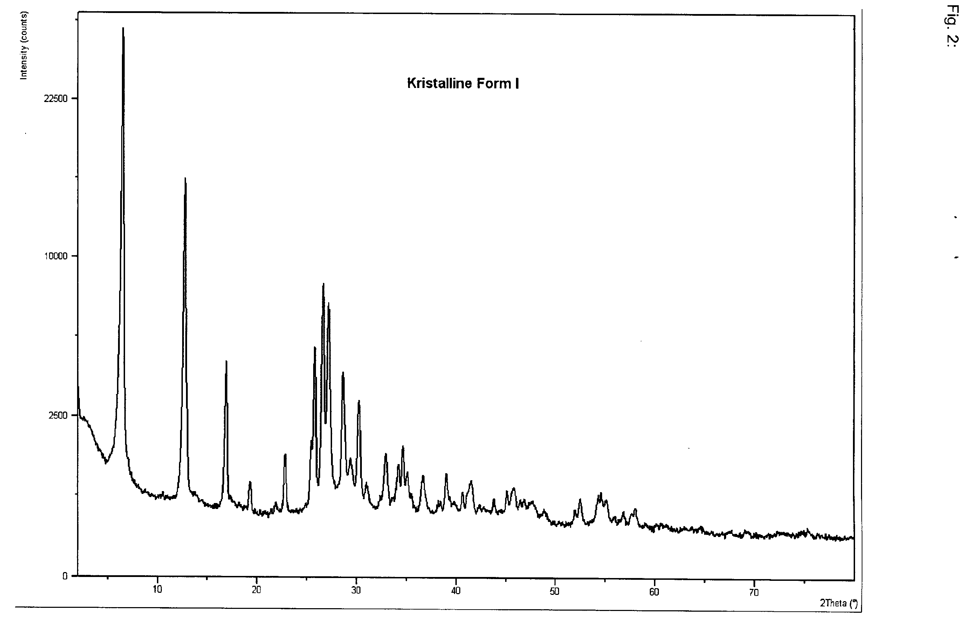

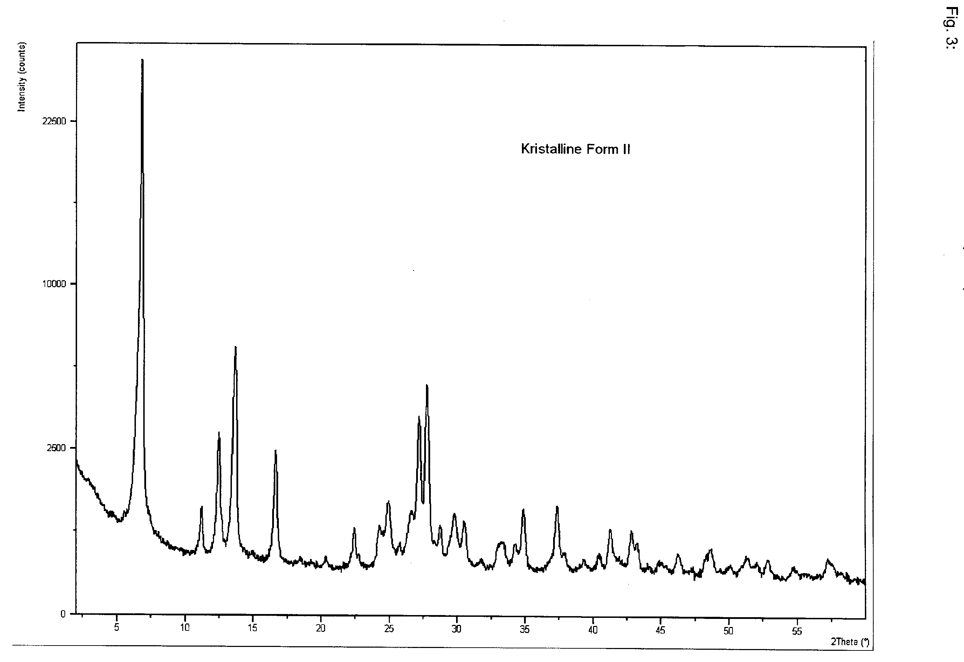

Kristalline Form I und Form II sind definiert durch je 10 charakteristische Werte zu Interplanarabständen und 2-Theta-Winkelwerten, ausgedrückt in je einem Röntgenpulverdiffraktogramm (Abb. 2 und 3).

Die Erfinder haben gefunden, dass die Formen I und II positive physikalische

Eigenschaften für die pharmazeutische Verarbeitung und Anwendung besitzen, einschließlich Stabilität, Lagerbeständigkeit, Nicht-Hygroskopizität und Löslichkeit. Diese sind vorteilhaft für die pharmazeutische Herstellung und Weiterverarbeitung im Vergleich zu beispielsweise Dihydraten, bei denen es zu Veränderungen im Wassergehalt und dadurch bedingt zu Formulierungsproblemen kommen kann, z.B. durch Gewichtsveränderungen des aktiven Wirkstoffs während der Tablettenpressung, Verkapselung oder Sterilisation. Unterschiede in physikalischen Eigenschaften bestehen zwischen den erfindungsgemäßen Formen und dem Stand der Technik beispielsweise in der Löslichkeit (vgl. Tabelle 6).

Die Erfinder haben es sich ferner zur Aufgabe gemacht, Verfahren bereitzustellen, welche die erfindungsgemäßen neuen Anhydratformen zu 5-Amino-2,3-dihydrophthalazin-1,4-dion Natriumsalz praktikabel und reproduzierbar ermöglichen. Die aufgezeigten Verfahren sollen ohne den Einsatz von Schwermetallkatalysatoren auskommen und die Herstellung der neuen kristallinen Form II auch für beliebige Ansatzgrößen reproduzierbar ermöglichen. Des Weiteren haben es sich die Erfinder zur Aufgabe gemacht, Verfahren zur Herstellung der kristallinen Formen I und II mit vorteilhaften Eigenschaften in Bezug auf die pharmazeutische Verarbeitung und die Anwendung in unterschiedlichen Applikationsarten bereit zu stellen.

Diese Aufgabe wurde durch mindestens ein Verfahren zur wahlweisen Herstellung kristalliner Form I oder II gelöst, bei dem Natriumhydroxid und Luminol in Wasser aufgelöst werden. Durch Zugabe eines niedermolekularen Alkohols, vorzugsweise Ethanol oder 2-Propanol, fällt kristallines Luminol Natriumsalz aus. Die gewünschte kristalline Form I oder II wird nach Isolierung und wiederholtem Waschen und Einhaltung einer bestimmten Rührdauer erhalten. Die erfindungsgemäßen Verfahren lassen sich für beliebige Ansatzmengen verwenden.

Als Ausgangsprodukt der Herstellung beider kristalliner Formen dient möglichst reines Luminol oder ein Verfahren zur Herstellung desselben durch Reduktion von 3-Nitrophthalsäure in alkalischem Medium mit einem geeigneten Reduktionsmittel über 3-Nitrophthalanhydrid. Optionale Aufreinigungsschritte durch Rekristallisation schließen sich an.

Die vorliegende Erfindung umfasst schließlich pharmazeutische Zubereitungen der Formen I oder II oder einer Kombination hiervon, jeweils alleine oder zusammen mit pharmazeutisch geeigneten Hilfsstoffen.

### KURZE BESCHREIBUNG DER FIGUREN:

Fig. 1a zeigt rasterelektronische Aufnahmen von Form I.
Fig. 1b zeigt rasterelektronische Aufnahme von Form II.
Fig. 2 beschreibt ein Pulverdiffraktogramm der kristallinen Form I.
Fig. 3 beschreibt ein Pulverdiffraktogramm der kristallinen Form II.
Fig. 4 beschreibt die Auflöserate von Form I und II in Wasser über die Zeit (20 min). Form I ist durch die untere der beiden Linien dargestellt, Form II durch die obere.

### DETAILLIERTE BESCHREIBUNG

Wenn nicht anders dargestellt, kommt den in der vorliegenden Erfindung verwendeten technischen und wissenschaftlichen Begriffen die Bedeutung zu, die ihnen der Fachmann im relevanten technischen Gebiet beimisst.

Ein "Organismus" ist ein Lebewesen, insbesondere Mensch oder Tier, das mit einem selbstregulierenden immunologischen System ausgestattet ist.

Der Begriff "Wirkstoff" umfasst die kristalline Form I oder die kristalline Form II oder eine Mischung aus beiden.

Der Begriff "pharmazeutische Zubereitung" umfasst den Wirkstoff in jeder pharmakologisch geeigneten, definierten Dosierung und Darreichungsform, wie z.B. als Pulver, Suspension, Emulsion und/oder Mischungen daraus. Er umfasst pharmazeutisch geeignete Hilfsstoffe, sowie alle Stoffe, welche direkt oder indirekt als eine Kombination, Anhäufung, Komplexbildung der Inhaltsstoffe entstehen, oder als Folge sonstiger Reaktionen oder Interaktionen sowie ferner weitere, andere aktive Wirkstoffe allein oder in Kombination.

Pharmazeutische Zubereitungen des erfindungsgemäßen Wirkstoffs, einzeln oder in Kombination mit anderen Adjuvanzien und Standardtherapien, können erfindungsgemäß in flüssiger und fester Form formuliert und in jeder medizinisch akzeptablen Weise verabreicht werden, vor allem, aber nicht ausschließlich, intravenös, intramuskulär, topisch (z. B. Augentropfen), subkutan, transdermal, vaginal, rektal oder oral, einschließlich sublingual und bukkal, sowie in Form von Substanz-eluierenden Implantaten. Flüssige Formen können sein: z.B. Lösungen (z.B. für Injektionen und Infusionen), Suspensionen, Emulsionen, Sprays, Lotionen und Salben). Feste Formen können sein: Tabletten, Dragees, Kapseln, Pulver oder weitere, dem Fachmann geläufige und als geeignet erscheinende Formen, z.B. Suppositorien.

Der Ausdruck "Hilfsstoff" wird hierin verwendet, um jeden Bestandteil einer pharmazeutischen Zubereitung neben dem Wirkstoff selbst zu beschreiben. Die Wahl eines geeigneten Hilfsstoff hängt von Faktoren wie Applikationsart und Dosierung ab sowie von der Beeinflussung der Löslichkeit und Stabilität der Zubereitung durch den Hilfsstoff selbst.

Pharmazeutische "Hilfsstoffe" sind Stoffe, die dem Fachmann bekannt oder Standardwerken der Pharmazeutik oder offiziellen Arzneibüchern (z.B. Europäische Pharmakopoe) zu entnehmen sind. Solche Stoffe können Beispiel Einfluss auf die Verteilung des Wirkstoffs in unterschiedlichen Geweben und Organen nehmen können oder die Wirkdauer oder Wirkgeschwindigkeit von Arzneiformen verändern, etwa durch Beschleunigung der Resorption (beispielsweise durch Dimethylsulfoxid, Nikotinsäure, Hyaluronidase) oder indem sie durch geeignete Retard-Präparate in ihrem Wirkungseintritt verzögert werden.

Pharmazeutische Hilfsstoffe zur Anwendung in der je nach Anwendungsart gewünschten Applikationsform können beispielsweise sein: Natriumcitrat, Calciumphosphat, Calciumcarbonat zusammen mit geeigneten Tablettensprengmitteln, z.B. bei oraler Applikation. Als solche kommen etwa Substanzen in Betracht, die durch Wasseraufnahme quellen (Stärke, Cellulose-Derivate, Alginate, Polysaccharide, Dextrane, quervernetztes Polyvinylpyrrolidon), Substanzen, die durch eine chemische Reaktion mit Wasser Gas entwickeln (Natriumhydrogencarbonat, Zitronen- und Weinsäure) oder Substanzen, welche als Hydrophylierungsmittel die Benetzung der Kristallite verbessern und damit ihre Auflösung im Wasser vermitteln (z. B. Polyethylenglycolsorbitanfettsäureester).

Hilfsstoffe sind auch Substanzen, die als Bindemittel eingesetzt werden können, wie z.B. Stärke, Gelatine, Zuckerstoffe, Cellulosederivate, oder als Verdünner, z.B. Zuckerstoffe. Ferner oberflächenaktive Substanzen, z.B. Natriumlaurylsulfat oder Polysorbat 80, oder Gleitmittel, wie z.B. Magnesiumstearat, Natriumstearat sowie ferner dem Fachmann als geeignet erscheinende Geschmacksstoffe, Antioxidantien, Färbemittel und Konservierungsstoffe.

Der Begriff "substanziell rein" bedeutet eine Reinheit des Wirkstoffs von mindestens 95%, bevorzugt 98%, höchst bevorzugt 99% 5-Amino-2,3-dihydrophthalazin-1,4-dion Natriumsalz.

Der Begriff "immunspezifisch" bedeutet die gezielte Verwendung von Form I und/oder Form II zur Behandlung von Krankheiten mit immundefizientem Hintergrund oder überschießendem Immunsystem.

Der Begriff "Wirkung" beschreibt die spezifische, hier immunspezifische, Wirkweise eines Wirkstoffs im Rahmen dieser Erfindung mit überwiegend immunstimulierender oder überwiegend immunsupprimierender Wirkung.

### Neue kristalline Formen I und II zu 5-Amino-2,3-dihydrophthalazin-1,4-dion Natriumsalz

Die vorliegende Erfindung beinhaltet eine neue kristalline Anhydratform I von 5-Amino-2,3-dihydrophthalazin-1,4-dion Natriumsalz gekennzeichnet durch ein Röntgenpulverbeugungsdiagramm eines mit einer Wellenlänge von lambda= 1,54187 Å, sowie mit X'Celerator Scientific RTMS Detektor ausgestattetem Bragg-Brantano-Diffraktometers (Panalytical X'Pert Pro) unter Verwendung einer mit Nickel gefilterten Kupferstrahlung Cu K(α1), das in D - oder 2-Theta-Werten ausgedrückt ist, wobei mit "D" die Interplanarabstände (Tabelle 1) und mit "2-Theta" die 2-Theta-Winkel in Grad bezeichnet sind. I (rel) gibt die relativen Intensitäten der Reflexe wieder (Tabelle 2):

**Tabelle 1: D-Werte der Anhydratform I:**

| | 13,51 | 6,94 | 5,24 | 4,59 | 3,89 | 3,45 | 3,35 | 3,28 | 3,11 | 2,95 |
|---|---|---|---|---|---|---|---|---|---|---|
| gerundete Werte | 13,5 | 6,9 | 5,2 | 4,6 | 3,9 | 3,5 | 3,4 | 3,3 | 3,1 | 3,0 |

**Tabelle 2: 2-Theta-Werte der Anhydratform I und relative Intensitäten I(rel)**

| 2Θ | 6,5 | 12,7 | 16,9 | 19,3 | 22,8 | 25,8 | 26,6 | 27,2 | 28,7 | 30,3 |
|---|---|---|---|---|---|---|---|---|---|---|
| I/Iₒ (rel) | vst | st | m | w | w | m | st | st | m | m |

| | | | | | | | | | | |
|---|---|---|---|---|---|---|---|---|---|---|
| , wobei gilt: w= weak, m= medium, st= strong und vst= very strong. | | | | | | | | | | |

Die vorliegende Erfindung beinhaltet ferner eine neue kristalline Anhydratform II von 5-Amino-2,3-dihydrophthalazin-1,4-dion Natriumsalz gekennzeichnet durch ein Röntgenpulverbeugungsdiagramm eines mit einer Wellenlänge von lambda= 1,54187 Å, sowie mit X'Celerator Scientific RTMS Detektor ausgestattetem Bragg-Brantano-Diffraktometers (Panalytical X'Pert Pro) unter Verwendung einer mit Nickel gefilterten Kupferstrahlung Cu K(α1), das in D - oder 2-Theta-Werten ausgedrückt ist, wobei mit "D" die Interplanarabstände (Tabelle 3) und mit "2-Theta" die 2-Theta Winkel in Grad bezeichnet sind. I (rel) gibt die relativen Intensitäten der Reflexe wieder (Tabelle 4).

**Tabelle 3: D-Werte der Anhydratform II:**

| | 12,92 | 7,88 | 7,08 | 6,47 | 5,32 | 3,96 | 3,66 | 3,57 | 3,27 | 3,21 |
|---|---|---|---|---|---|---|---|---|---|---|
| gerundete Werte | 12,9 | 7,9 | 7,1 | 6,5 | 5,3 | 4,0 | 3,7 | 3,6 | 3,3 | 3,2 |

**Tabelle 4: 2-Theta-Werte der Anhydratform II und relative Intensitäten I(rel)**

| 2Θ | 6,8 | 11,2 | 12,5 | 13,7 | 16,7 | 22,4 | 24,3 | 24,9 | 27,2 | 27,8 |
|---|---|---|---|---|---|---|---|---|---|---|
| I (rel) | vst | w | m | st | m | w | w | w | m | st |

| | | | | | | | | | | |
|---|---|---|---|---|---|---|---|---|---|---|
| , wobei gilt: w= weak, m= medium, st= strong und vst= very strong. | | | | | | | | | | |

### Vorteilhafte physikalische Eigenschaften:

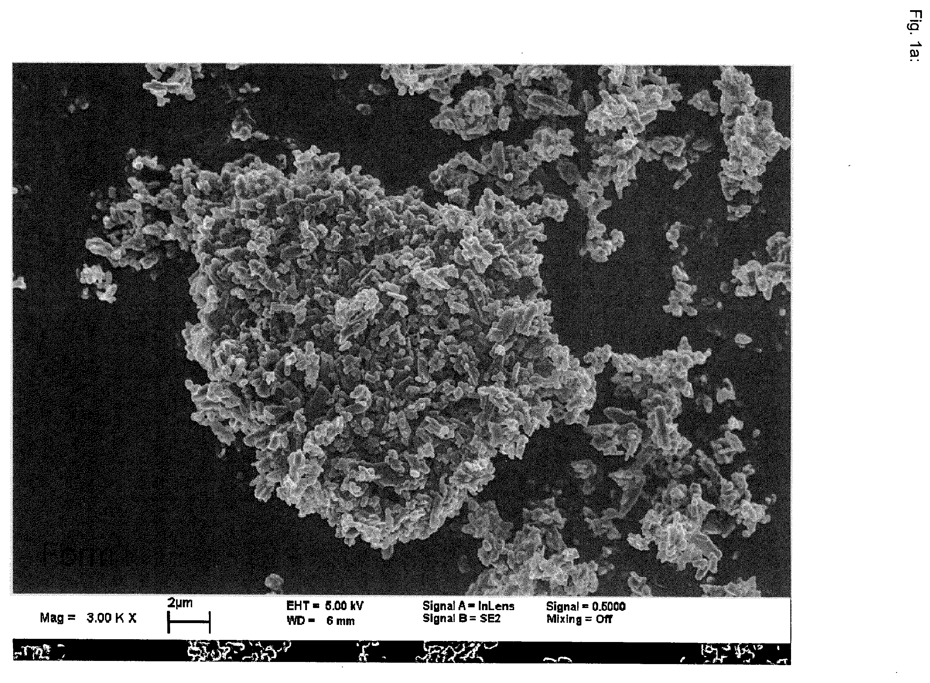

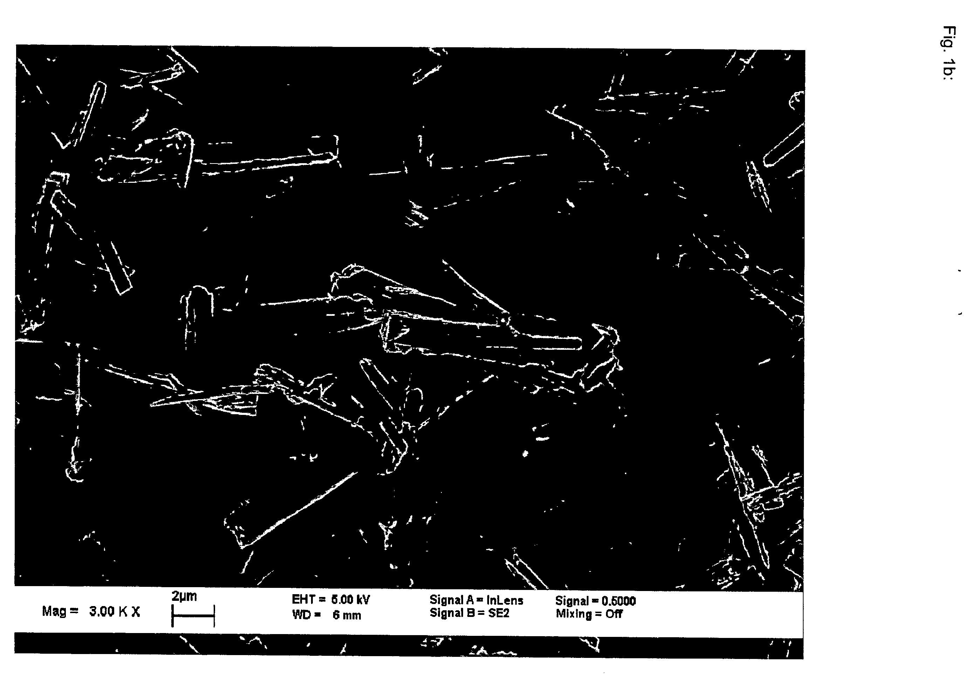

Beide erfindungsgemäßen Formen lassen sich rasterelektronenmikroskopisch voneinander unterscheiden: So weist Form II vor allem nadelähnliche Kristallite oktaedrischer Struktur von mehreren Mikrometern Länge auf, die aus Schichten aufgebaut sind, während sich im REM der Form I größtenteils morphologisch uneinheitliche Kristallite mit abgerundeten Kanten zeigen, die pulverförmig agglomerieren (Abb. 1). Aus ihrer Kristallform ergeben sich für die erfindungsgemäßen Formen vorteilhafte Eigenschaften für die pharmazeutische Verarbeitung: So weist Form II gegenüber Form I aufgrund seiner Kristallpartikelform eine höhere Rieselfähigkeit und damit eine verbesserte Filtrierbarkeit auf, wohingegen sich Form I insbesondere aufgrund seiner höheren Schüttdichte besser für eine Tablettenverpressung eignet, was durch seine Neigung zur Agglomeration begünstigt wird, möglicherweise infolge des Verklebens seiner laminaren Substrukturen.

Beide kristalline Formen sind über einen Zeitraum von mindestens zwei Monaten stabil bei Raumtemperatur (25 °C) und 40 °C und zersetzen sich erst ab 335°C ± 10°C (Form I) bzw. 385°C ± 10°C (Form II), während bei dem Dihydrat nach US 6,489,326 B1 bereits bei 85°C eine endotherme Festphasenumwandlung zu beobachten ist (Tabelle 5). Die Festphasenumwandlung des Dihydrats nach US 6,489,326 B1 wurde mit Hilfe der simultanen Thermogravimetrie - Differential-Thermoanalyse an einem Linseis L81-077 gekoppelt mit Massenspektroskopie-Messungen mit einer Netzsch STA 449 C, Thermowaage, mit MS- und FTIR-Kopplung über 30-300 °C unter Helium, gemessen. Die Zersetzungstemperaturen der Formen I-II wurden mit demselben Gerät über 30-500°C in synthetischer Luft (4 N₂ : 1 O₂) bestimmt. Die Daten wurden mit der werksseitigen Software Proteus ausgewertet.

**Tabelle 5: Zersetzungstemperaturen Form I-II und die Bestimmung der Festphasenumwandlung des Dihydrats nach US 6,489,326 B1**

| Form I | Form II | Dihydrat US 6,489,326 B1 |
|---|---|---|
| 335 °C ± 10 °C | 385 °C ± 10 °C | 85 °C ± 10°C |

Die thermoanalytischen Daten bestätigen die Annahme der Erfinder, dass beide kristalline Formen vorteilhafte Eigenschaften in Bezug auf Stabilität und Lagerungsbeständigkeit aufweisen. Diese Eigenschaften begünstigen ferner die pharmazeutische Verarbeitung der erfindungsgemäßen kristallinen Formen I und II gegenüber dem Dihydrat der US 6,489,326 B1, indem sie diese gegenüber Verfahrensschritten mit hohem Energieeintrag, z.B. Sterilisation oder Vermahlung, unempfindlich machen.

Darüber hinaus zeigen sich beide kristalline Formen I und II substantiell stabil in Bezug auf eine Veränderung des Wassergehalts, sodass Formulierungsprobleme infolge Veränderungen des Wirkstoffgewichts bei der pharmazeutischen Weiterverarbeitung (z.B. Tablettierung, Verkapselung etc.) reduziert sind.

Ferner wurde die Löslichkeit der kristallinen Formen in einer gesättigten Lösung bestimmt. Danach ist Form II leichter löslich als Form I; beide Formen sind ihrerseits deutlich leichter löslich als das Dihydrat nach US 6,489,326 B1. Die neuen kristallinen Formen stellen einen Vorteil gegenüber dem Stand der Technik dar, da eine höhere und gute maximale Löslichkeit bei Injektionslösungen kleinere Injektionsvolumina ermöglicht und bei topischen Zubereitungen, wie Cremes mit einem geringen Wasseranteil eine deutlich bessere Verarbeitbarkeit bedeutet.

**Tabelle 6: Übersicht Löslichkeit von Form I und Form II in Wasser bei Raumtemperatur im Vergleich mit Dihydrat-US 6,489,326 B1 herstellbar nach US 6,489,326 B1**

| Form I | Form II | Dihydrat/US 6,489,326 B1 |
|---|---|---|
| 227 mg/mL | 252 mg/mL | 168 mg/mL |

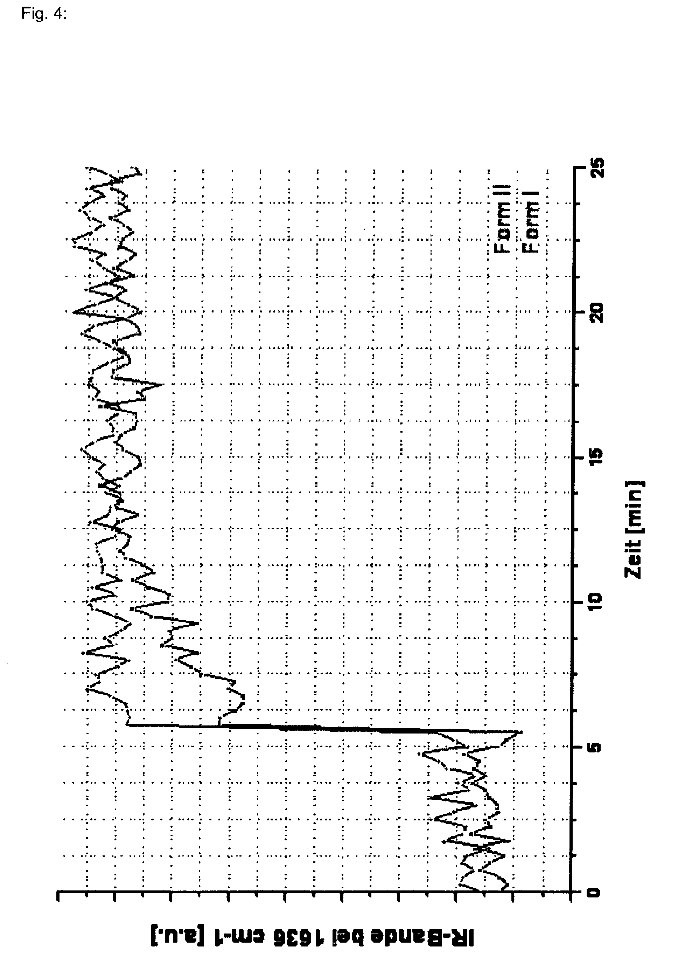

Die vergleichbar bessere Löslichkeit der Form II wird auch von den Daten zur intialen Auflöserate unterstützt. Diese Daten stammen aus einer in-situ ATR-IR-Messung in Messintervallen von je 15 s, wobei die Formen I und II 5 min nach Aufzeichnungsbeginn zugegeben wurden. Die vollständige Lösung von 0,25 g Form I bzw. Form II in 10 mL H2O VE wurde unter Rühren (500 rpm) bei 25° C erreicht. Es zeigte sich, dass die Auflöserate von Form II in den ersten Minuten nach Zugabe zu einer wässrigen Lösung höher liegt als bei Form I, die erst verzögert ihre vollständige Gleichgewichtskonzentration erlangt. Die unterschiedlichen Löslichkeiten sowie initialen Auflöseraten der Formen I und II (Abb. 4) könnten in deren unterschiedlichen Oberflächenstrukturen begründet sein (Abb. 1). Die morphologisch uneinheitlichen abgerundeten Kristallite der Form I stellen eine tendenziell kompaktere Oberflächenstruktur dar als die oktaedrischen Kristalle der Form II, die aufgrund ihrer Form Lösungsmitteln eine größer zugängliche Oberfläche bieten. Die Unterschiede in der initialen Auflöserate zwischen Form I und II sind insbesondere für die Herstellung oraler Formulierungen von Bedeutung, da eine langsamere Auflöserate wie bei Form I dort Vorteile bietet, wo eine verzögerte Wirkstoff-Freisetzung angestrebt wird (Retard-Formulierungen). Eine schnellere Auflöserate wie bei Form II ist für die Formulierung oraler Akutmedikationen vorteilhaft, bei denen eine möglichst schnelle und hohe Bioverfügbarkeit angestrebt wird.

Die unterschiedlichen thermischen Stabilitäten der Formen I und II begründen sich in der unterschiedlichen stöchiometrischen Koordination des Natrium-Kations und der Luminolat-Moleküle. Während in der Form I ein Natrium-Kation über intermolekulare Wasserstoffbrückenbindungen insgesamt 6 Luminolatmoleküle in einem trigonalen Prisma koordiniert, sind es in der Form II nur 4 Luminolatmoleküle, die tetraedisch über intermolekulare Wasserstoffbrückenbindungen angeordnet sind. Daraus ergibt sich eine thermisch tendenziell günstigere und stabilere Koordination der Form II gegenüber Form I.

### Medizinische Verwendung:

Überraschenderweise fanden die Erfinder in experimentellen in vitro und in vivo-Studien eine erkennbar unterschiedliche immunologische Wirkung zwischen der kristallinen Form I und II.

Ferner wurde gefunden, dass sich die Formen I und II aufgrund ihrer unterschiedlichen immunmodulatorischen Wirkung spezifischer über dem Stand der Technik einsetzen lassen: Während Form II eine immunmodulierende überwiegend supprimierende Wirkung auf bestimmte Zytokine zeigt, zeigt Form I eine überwiegend immunstimulierende Wirkung auf bestimmte Zytokine. Form II eignet sich daher insbesondere zur therapeutischen Anwendung bei Zuständen mit überschießenden Immunreaktionen, Form I eignet sich insbesondere zur therapeutischen Anwendung bei Indikationen mit immundefizientem Hintergrund.

Gerade bei chronischen Erkrankungen (z.B. Multiple Sklerose, Hepatitis C, chronische Enteritiden und Colitiden) kann sich der Immunstatus der Patienten immer wieder derart verändern, dass ein Wechsel von immunstimulierenden auf immunsupprimierende Therapien oder von immunsupprimierend auf immunstimulierend oder eine kombinierte oder zeitversetzte Gabe beider Ansätze sinnvoll erscheint (vgl. DMSG 2006: Aktuelle Therapieempfehlungen September 2006). Daher können die folgenden Aufzählungen nur beispielhaft betrachtet werden, die Zuordnung einer Krankheit zu Zuständen mit überschießenden Immunreaktionen schließt nicht deren Behandlung mit Form II oder mit Kombinationen von Form I und Form II aus und die Zuordnung einer Krankheit zu Zuständen mit immundefizientem Hintergrund schließt nicht deren Behandlung mit Form I oder mit Kombinationen von Form I und Form II aus.

Als Zustände mit überschießenden Immunreaktionen seien beispielhaft genannt Abstoßungsreaktionen nach Transplantationen, aktive Autoimmunerkrankungen (insbesondere aktive Rheumatoide Arthritis, schubförmige Multiple Sklerose, lupoide Hepatitis, Polyarteriitis nodosa, Morbus Crohn, Colitis ulcerosa, Dermatomyositis. Morbus Behcet, Morbus Behcet Uveitis, thrombozytopenische Purpura, Myasthenia gravis, Polymyositis, Psoriasis, Psoriasis Arthritis, Morbus Bechterew, paroxysmale nächtliche Hämoglobinurie, ankylosierende Spondylitis, Autoimmun-Thyreoiditis, etc.), aplastische Anämie, Pemphigus, Pemphigoid, endogene Uveitis, nephrotisches Syndrom und atopische Dermatitis und besonders bevorzugt septische Zustände ausgelöst durch bakterielle Infektionen mit gram-negativen oder gram-positiven Erregern, wie z.B. durch MRSA (Methicillin-resistenter Staphylokokkus aureus) und Systemisches inflammatorisches Response-Syndrom (SIRS) ausgelöst durch andere (z.B. immunologische oder chemische) Faktoren.

Als Zustände mit immundefizientem Hintergrund seien beispielhaft genannt häufige grippale Infekte, rezidivierende Atemwegsinfekte, rezidivierende Infekte der ableitenden Harnwege, Müdigkeit, Schwäche, Konzentrationsstörungen unbekannter Genese, Rekonvaleszenz, chronische Virusinfektionen (insbesondere HIV, Hepatitis B, Hepatitis C, Enzephalitis, Herpes zoster, Herpes simplex, Innenohrinfekte, Varizellen, Masern, Zytomegalie, Epstein-Barr), verschiedene onkologische Erkrankungen (insbesondere Haarzellenleukämie, Myeloische Leukämie, Multiples Myelom, Follikuläre Lymphome, Kaposi-Sarkom, Kutanes T-Zell-Lymphom, Nasopharynxkarzinom, Karzinoid, Nierenkarzinom, Harnblasenkarzinom, Basalzellkarzinome, metastasierende Karzinome und besonders bevorzugt Malignes Melanom), septische Granulomatose, Neutropenie, Feigwarzen, Keratosen, Autoimmunerkrankungen (insbesondere nicht aktive Stadien, wie zum Beispiel schubförmige Multiple Sklerose zwischen den Schüben), Radiogene Colitis, Divertikelkrankheit, Allergien (insbesondere Heuschnupfen, Polymorphe Lichtdermatose, Ekzem, Neurodermitis), Enteritis, Colitis, sowie besonders bevorzugt begleitend vor, während und nach Chemo- und Strahlentherapien.

Die Erfinder konnten überraschenderweise durch die einfache Bereitstellung von spezifischen Kristallformen des 5-Amino-2,3-dihydrophthalazin-1,4-dion Natriumsalzes, bevorzugt von Polymorphen des Anhydrats, besonders bevorzugt von Form I und Form II, unterschiedliche Wirkungen zeigen. Diese erlauben eine gezielte immunmodulatorische Anwendung zu vorwiegend immunstimulierenden (Form I) oder vorwiegend immunsupprimierenden (Form II) Zwecken, wobei beide Formen generell regulierend auf das Immunsystem wirkten. So konnten die Erfinder in vitro und in vivo einen überraschenden immunstimulierenden Effekt für Form I nachweisen.

Form II zeigt dagegen immunmodulatorische Eigenschaften, die sich vorteilhaft auf aktivierte Makrophagen auswirken. Die Erfinder konnten insbesondere vorwiegend immunsupprimierende Eigenschaften von Form II anhand von in vitro-Versuchen mit LPSstimulierten Makrophagen belegen. Hierbei kam es zu einer deutlichen Senkung der IL-6-Werte. Bei in vivo-Versuchen an Mäusen (Sepsismodell mit S. pyogenes) konnte ebenfalls eine deutliche therapeutische Wirksamkeit von Form II nachgewiesen werden.

Weiterhin konnten die Erfinder anhand der Gewichtsentwicklung und der Leberenzymwerte gesunder Mäuse zeigen, dass die neuen Formen I und II keine beziehungsweise nur eine geringe Toxizität aufweisen. Beide Formen wirken immunmodulatorisch ohne dabei ausschließlich immunstimulierend oder ausschließlich immunsupprimierend zu wirken. Sowohl Form I als auch Form II führen im Sepsismodell zu höheren Überlebensraten.

Die Erfinder gehen davon aus, dass auch eine kombinierte, bevorzugt zeitversetzte, Anwendung von Form I und Form II erfolgsversprechend sein kann, und dass eine kombinierte Anwendung für bestimmte Indikationen, insbesondere aber nicht ausschließlich Autoimmunerkrankungen, vorteilhaft gegenüber der Einzelanwendung sein kann.

### Beispiel 1 - Wirkung von Form I auf LPS-stimulierte murine Makrophagen in vitro

In einem Versuch mit isolierten Mausmakrophagen sollte in vitro die immunmodulatorische Wirkung der Form I anhand der in den Überständen gemessenen Konzentration von TNF-alpha oder IL-6 aufgezeigt werden. Dazu wurden die Makrophagen zunächst mit Form I (20 oder 200 µg/mL) behandelt. Nach 1 Stunde wurden die behandelten Makrophagen mit LPS (10, 100 oder 1000 ng/mL) stimuliert. Die Überstände wurden 24 h später gesammelt und die Konzentration an TNF-alpha und IL-6 gemessen. Als Kontrollen dienten nicht-stimulierte Makrophagen, nicht-stimulierte Makrophagen behandelt mit 200 µg/mL Form I sowie LPS-stimulierte Makrophagen (10, 100 oder 1000 ng/mL LPS). Die gemessenen Werte lassen sich der Tabelle 7 entnehmen.

Die TNF-alpha-Messwerte aus Tabelle 7 zeigen bei alleiniger Gabe von LPS (in Konzentrationen von 1 µg/mL, 100 ng/mL und 10 ng/mL) eine dosisabhängige Stimulierung durch LPS.

**Tabelle 7:**

| | Dosis | LPS | N | TNF-alpha (ng/mL) | | IL-6 (ng/mL) | |
|---|---|---|---|---|---|---|---|
| | | | | MW | STD | MW | STD |
| Form I | 20 µg | 1 mg/mL | 6 | 47,91 | 3,89 | 22,67 | 2,21 |
| | 200 µg | 1 mg/mL | 6 | 32,53 | 4,44 | 15,95 | 3,84 |
| | 20 µg | 100 ng/mL | 6 | 27,17 | 4,77 | 19,67 | 3,76 |
| | 200 µg | 100 ng/mL | 6 | 16,47 | 4,07 | 10,88 | 2,09 |
| | 20 µg | 10 ng/mL | 6 | 13,27 | 5,99 | 6,05 | 4,30 |
| | 200 µg | 10 ng/mL | 6 | 20,74 | 7,57 | 7,19 | 5,56 |
| Kontrolle I (nicht-stimuliert) | | | 3 | 0,00 | 0,00 | 0,00 | 0,00 |
| Kontrolle II (200 µg/mL Form I) | 200 µg/mL | | 6 | 7,98 | 6,53 | 0,24 | 0,41 |
| Kontrolle III (1 µg/mL LPS) | | 1 µg/mL | 3 | 50,97 | 6,59 | 28,62 | 2,07 |
| Kontrolle III (100 ng/mL LPS) | | 100 ng/mL | 3 | 32,32 | 2,10 | 26,97 | 4,42 |
| Kontrolle III (10 ng/mL LPS) | | 10 ng/mL | 3 | 25,36 | 5,61 | 27,70 | 4,02 |

In den Kombinationsgruppen mit LPS und mit Form I behandelten Makrophagen zeigte sich über alle LPS-Gruppen hinweg ein dosisabhängiger, immunsupprimierender Effekt der Form I. Dieser fiel in der Kombination mit 100 ng/mL LPS stimulierten und mit 200 µg/mL Form I behandelten Makrophagen am deutlichsten aus, gefolgt von einer signifikanten Reduktion an TNF-alpha -Werten in der Kombination von 1 µg/mL LPS mit 200 µg/mL Form I.

Außerdem ließ sich in der Kontrollgruppe der nicht-stimulierten, mit 200 µg/mL Form I behandelten Makrophagen ausweislich der gemessenen TNF-alpha-Werte ein deutlicher immunmodulatorischer, hier immunstimulierender Effekt beobachten.

### Beispiel 2 - Wirkung von Form II auf LPS-stimulierte murine Makrophagen in vitro

In einem weiteren in vitro-Modell mit Knochenmarksmakrophagen (Maus) ließ sich der immunmodulatorische, überwiegend immunsupprimierende Behandlungseffekt der Form II bestätigen. Dazu wurden isolierte Maus-Knochenmarksmakrophagen zunächst mit Form II in Konzentrationen von je 2, 20 oder 200 µg/mL behandelt und 1 h nach Behandlung mit 100 ng/mL oder 10 ng/mL LPS stimuliert. Die Überstände wurden 24 h später gesammelt und die Konzentration an TNF-alpha und IL-6 gemessen. Als Kontrolle dienten nicht-stimulierte Makrophagen, nicht-stimulierte Makrophagen behandelt mit 200 µg/mL Form II sowie LPS-stimulierte Makrophagen (10 oder 100 ng/mL LPS). Die gemessenen Werte lassen sich der Tabelle 8 entnehmen.

**Tabelle 8:**

| | Dosis | LPS | N | TNF-alpha (ng/mL) | | IL-6 (ng/mL) | |
|---|---|---|---|---|---|---|---|
| | | | | MW | STD | MW | STD |
| Form II | 2 µg | 100 ng/mL | 7 | 7,40 | 3,49 | 2,67 | 0,76 |
| | 20 µg | 100 ng/mL | 9 | 9,06 | 1,36 | 4,28 | 0,54 |
| | 200 µg | 100 ng/mL | 9 | 7,69 | 1,62 | 4,00 | 1,20 |
| | 2 µg | 10 ng/mL | 9 | 8,38 | 1,76 | 3,93 | 1,01 |
| | 20 µg | 10 ng/mL | 9 | 9,80 | 0,72 | 1,61 | 1,30 |
| | 200 µg | 10 ng/mL | 6 | 6,76 | 1,60 | 3,42 | 1,29 |
| Kontrolle (nicht-stimuliert) | | | 9 | 0,00 | 0,00 | 0,00 | 0,00 |
| Kontrolle (nicht stimuliert) | 200 µg | | 9 | 0,00 | 0,00 | 0,00 | 0,00 |
| Kontrolle | | 100 ng/mL | 9 | 12,46 | 1,70 | 4,47 | 1,67 |
| Kontrolle | | 10 ng/mL | 9 | 9,45 | 1,47 | 3,19 | 1,33 |

Eine signifikante Reduzierung der TNF-alpha-Konzentration ließ sich in der Gruppe mit 100 ng/mL LPS stimulierten Makrophagen in allen Wirkstoffkonzentrationen messen. In der Gruppe der mit 10 ng/mL LPS stimulierten Makrophagen zeigte sich eine signifikante Reduzierung an TNF-alpha in der höchsten Wirkstoffkonzentration (200 µg/mL).

Ein immunsupprimierender Effekt der Form II auf LPS-stimulierte Makrophagen konnte außerdem anhand von signifikanten Reduzierungen bei IL-6-Werten beobachtet werden. Diese wurden gemessen in der Kombination von 2 µg/mL Wirkstoff mit 100 ng/mL LPS stimulierten Makrophagen sowie in der Gruppe der mit 10 ng/mL stimulierten Makrophagen in der Wirkstoffkonzentration mit 20 µg/mL. Diese Ergebnisse stehen im Einklang mit IL-6-Werten aus einem früheren in vitro-Pilotversuch mit Makrophagen, in welchem eine andere LPS- und Wirkstoffkombination (1 µg/mL LPS mit 100 µg/mL) verwendet wurde und der ebenfalls einen immunsupprimierenden Effekt von Form II auf stimulierte murine Makrophagen zeigte. Ein immunstimulierender Effekt auf nicht LPSinduzierte Makrophagen konnte nicht gezeigt werden.

### Beispiel 3 - Gewichtsentwicklung bei gesunden Mäusen

In einem Mausmodell der gram-positiven Sepsis (Infektion mit S. pyogenes) wurde die therapeutische Einsatzfähigkeit der Kristallformen I und II des 5-Amino-2,3-dihydrophthalazin-1,4-dion Natriumsalzes geprüft. Die Wirkstoffgabe erfolgte parenteral 6 Stunden nach erfolgter Infektion. Eine zweite Wirkstoffgabe erfolgte 24 Stunden nach der ersten Applikation. Der Versuch wurde 48 Stunden nach der Infektion beendet. Für beide Formen (Substanzgruppen) kamen drei verschiedene Dosierungen zum Einsatz. Die Mäuse erhielten pro Tier und Applikation jeweils 2, 20 oder 200 µg von Form I oder Form II.

Neben den Substanzgruppen (Form I und Form II) erhielt eine Gruppe Kochsalzlösung (NaCl) anstelle der gelösten Wirksubstanzen und eine Gruppe erhielt gar keine Behandlung. Parallel zu den infizierten Tieren wurden vergleichbare Gruppen mit nicht infizierten Tieren gebildet.

Die einzelnen Behandlungsgruppen setzten sich aus jeweils 5 Tieren zusammen, wobei für die nicht infizierten Kontrollen zwei und für die infizierten Kontrollen sowie jene Gruppen die Form II erhielten vier Wiederholungen erfolgten, so dass sich insgesamt 180 Tiere im Versuch befanden.

Es ist bekannt, dass sich die Toxizität von Substanzen im Tierversuch bei heranwachsenden Mäusen oder Ratten auf die Gewichtsentwicklung auswirken kann. Die Gewichtsentwicklung (g) der nicht infizierten Kontrolltiere über einen Zeitraum von zwei Tagen ist in Tabelle 9 zusammengefasst.

**Tabelle 9**

| | Dosis | N | Gewichtsentwicklung/Zunahme (g) | |
|---|---|---|---|---|
| | | | MW | STD |
| Form I | (gesamt) | 15 | 0,59 | 1,33 |
| | 2 µg | 5 | -0,49 | 0,81 |
| | 20 µg | 5 | 1,48 | 1,58 |
| | 200µg | 5 | 0,79 | 0,73 |
| Form II | (gesamt) | 30 | 0,59 | 2,19 |
| | 2 µg | 10 | -0,14 | 1,79 |
| | 20 µg | 10 | 1,43 | 2,14 |
| | 200 µg | 10 | 0,50 | 2,52 |
| NaCl | - | 10 | 0,67 | 1,87 |
| Kontrolle | - | 10 | 0,68 | 1,93 |
| Gesamt | - | 65 | 0,62 | 1,89 |

Unter den nicht infizierten Tieren gab es keine Todesfälle. Relevante Unterschiede in der Gewichtsentwicklung zwischen den Gruppen konnten nicht festgestellt werden. Daher ist davon auszugehen, dass sowohl Form I als auch Form II keine beziehungsweise nur sehr geringe Toxizität aufweisen.

### Beispiel 4 - Gewichtsentwicklung bei infizierten Mäusen

In dem in Bespiel 3 beschriebenen Sepsismodell wurde der Einfluss der Kristallformen I und II des 5-Amino-2,3-dihydrophthalazin-1,4-dion Natriumsalzes auf die Gewichtsentwicklung von mit S. pyogenes infizierten Mäusen geprüft. Die Entwicklung des Körpergewichts im Laufe einer akuten Infektion kann wichtige prognostische Hinweise liefern. Einige Tiere überlebten den zweiten Tag nicht bis Versuchsende. Tendenziell stellte die Körpergewichtsentwicklung des Individuums im Laufe der ersten 24 Stunden nach der Infektion einen Hinweis für die Wahrscheinlichkeit bis zum Versuchsende nach 48 Stunden zu überleben dar, ein signifikanter Zusammenhang bestand jedoch nicht. Da es für die im Laufe des zweiten Tages verendeten Tiere keine Daten zur Gewichtsentwicklung über die gesamte Versuchsdauer gibt, ist in der folgenden Tabelle 10 neben der Gewichtsentwicklung für die gesamte Versuchsdauer auch die Gewichtsentwicklung des ersten Versuchstages dargestellt.

**Tabelle 10:**

| | Dosis | Gewichtsentwicklung/Zunahme (g) | | | | | |
|---|---|---|---|---|---|---|---|
| | | Tag 1 | | | gesamt | | |
| | | N | MW | STD | N | MW | STD |
| Form I | (gesamt) | 15 | -0,25 | 0,39 | 12 | -1,85 | 0,54 |
| | 2 µg | 5 | -0,64 | 0,21 | 4 | -2,41 | 0,37 |
| | 20 µg | 5 | -0,09 | 0,31 | 4 | -1,46 | 0,28 |
| | 200 µg | 5 | -0,03 | 0,32 | 4 | -1,69 | 0,47 |
| Form II | (gesamt) | 60 | -0,62 | 1,56 | 48 | -1,86 | 1,65 |
| | 2 µg | 20 | -0,57 | 1,86 | 18 | -2,02 | 1,34 |
| | 20 µg | 20 | -0,55 | 1,56 | 16 | -1,69 | 0,85 |
| | 200 µg | 20 | -0,72 | 1,28 | 14 | -1,86 | 2,56 |
| NaCl | - | 20 | -0,68 | 1,43 | 14 | -1,77 | 1,89 |
| Kontrolle | - | 20 | -0,50 | 2,16 | 11 | -1,85 | 1,21 |
| Gesamt | - | 115 | -0,56 | 1,55 | 85 | -1,84 | 1,51 |

Tendenziell zeigten die Mäuse, die Form I des 5-Amino-2,3-dihydrophthalazin-1,4-dion Natriumsalzes erhalten, geringere Gewichtsverluste in den ersten 24 Stunden nach der Infektion als die Tiere in den Kontrollgruppen. Besonders deutlich wird dieser Vorteil bei den Dosierungen 20 und 200 µg von Form I. Dieser indirekte Hinweis auf eine tendenziell höhere Überlebensrate in den Substanzgruppen ist bei den Tieren, die den zweiten Tag überlebt haben, nicht mehr erkennbar.

### Beispiel 5 - Überlebenswahrscheinlichkeit nach S. pyogenes - Infektion

In dem in Beispiel 3 beschriebenen Sepsismodell wurde der Einfluss der Kristallformen Form II und Form I des 5-Amino-2,3-dihydrophthalazin-1,4-dion Natriumsalzes auf die Überlebenswahrscheinlichkeit der mit S. pyogenes infizierten Mäuse geprüft. Die einzelnen Gruppen zeigten unterschiedliche Wahrscheinlichkeiten mindestens 48 Stunden nach Infektion zu überleben. Die Anteile der überlebendenden Tiere sind in Tabelle 11 zusammengefasst.

**Tabelle 11:**

| | Dosis | N | Anteil überlebender Mäuse (%) |
|---|---|---|---|
| Form I | (gesamt) | 15 | 80 |
| | 2 µg | 5 | 80 |
| | 20 µg | 5 | 80 |
| | 200 µg | 5 | 80 |
| Form II | (gesamt) | 60 | 80 |
| | 2 µg | 20 | 90 |
| | 20 µg | 20 | 80 |
| | 200 µg | 20 | 70 |
| NaCl | - | 20 | 70 |
| Kontrolle | - | 20 | 55 |
| Gesamt | - | 115 | 74 |

Form I und Form II führten beide zu einer Erhöhung des Anteils überlebender Tiere gegenüber den Kontrollen. Somit ist sowohl für Form I als auch für Form II ein positiver Einfluss auf das septische Geschehen anzunehmen auch unabhängig von ihrer jeweiligen spezifischen Wirkung.

### Beispiel 6 - Keimbelastung in Blut und Leber

In dem in Beispiel 3 beschriebenen Sepsismodell wurde der Einfluss der Kristallformen Form II und Form I des 5-Amino-2,3-dihydrophthalazin-1,4-dion Natriumsalzes auf die Keimbelastung der Mäuse mit S. pyogenes in Blut und Leber geprüft. Der Versuch wurde 48 Stunden nach der Infektion beendet und die zu diesem Zeitpunkt bestehende Keimbelastung (KBE) in Blut und Leber eruiert (Tabelle).

**Tabelle 12:**

| | Dosis | Keimbelastung (KBE) | | | | | |
|---|---|---|---|---|---|---|---|
| | | Blut (log/mL) | | | Leber (log/g) | | |
| | | N | MW | STD | N | MW | STD |
| Form I | (gesamt) | 12 | 6,37 | 1,20 | 12 | 6,41 | 0,65 |
| | 2 µg | 5 | 7,00 | 1,49 | 4 | 6,56 | 0,97 |
| | 20 µg | 5 | 6,33 | 1,06 | 4 | 6,57 | 0,37 |
| | 200 µg | 5 | 5,77 | 0,94 | 4 | 6,09 | 0,55 |
| Form II | (gesamt) | 46 | 5,80 | 1,37 | 47 | 6,36 | 1,52 |
| | 2 µg | 16 | 5,96 | 1,21 | 17 | 6,13 | 1,69 |
| | 20 µg | 16 | 5,88 | 1,65 | 16 | 6,79 | 0,76 |
| | 200 µg | 14 | 5,54 | 1,26 | 14 | 6,14 | 1,91 |
| NaCl | - | 14 | 6,39 | 1,42 | 14 | 6,20 | 1,83 |
| Kontrolle | - | 2 10? | 6,50 | 1,03 | 10 | 6,86 | 0,55 |
| Gesamt | - | 82 | 6,07 | 1,33 | 83 | 6,40 | 1,40 |

Form II sowie Form I in der höchsten Dosierung zeigten eine tendenzielle Keimzahlreduktion im Blut im Vergleich zu den Kontrollen. Die Erfinder betrachten dies als Indiz für eine bessere Kontrollierbarkeit der Infektion durch die Gabe von 5-Amino-2,3-dihydrophthalazin-1,4-dion Natriumsalz, insbesondere Form 2. Einschränkend sei erwähnt, dass von vor Versuchsende verendeten Tieren keine Keimzahlbestimmungen vorliegen. Die Erfinder gehen davon aus, dass bei einer Keimzahlbestimmung zu einem früheren Zeitpunkt die Keimbelastung der später verendeten Tiere besonders hoch gewesen wäre und dadurch die durch die Formen I und II hervorgerufenen Effekte noch deutlicher in Erscheinung treten würden.

### Beispiel 7 - Zytokine bei gesunden Versuchstieren

Bei der Verabreichung von Form I und Form II an gesunde Mäuse wurde im Rahmen des in Beispiel 3 beschriebenen Tiermodells untersucht, ob und welchen Einfluss die Applikation auf bestimmte Zytokine, insbesondere IL-6 und TNF-alpha, in Mäusen zeigt. Die Blutentnahme erfolgte nach 48 Stunden am Ende des zweiten Anwendungstages. Für IL-6 konnten weder in den Substanzgruppen noch in den Kontrollgruppen Erhöhungen festgestellt werden. Die Ergebnisse für TNF-alpha sind in Tabelle 13 zusammengefasst.

**Tabelle 13:**

| | Dosis | N | TNF-alpha (pg/mL) | |
|---|---|---|---|---|
| | | | MW | STD |
| Form I | (gesamt) | 15 | 208,2 | 219,4 |
| | 2 µg | 5 | 128,8 | 137,2 |
| | 20 µg | 5 | 341,8 | 209,6 |
| | 200 µg | 5 | 154,1 | 268,2 |
| Form II | (gesamt) | 30 | 5,65 | 30,93 |
| | 2 µg | 10 | 0,0 | 0,0 |
| | 20 µg | 10 | 0,0 | 0,0 |
| | 200 µg | 10 | 16,94 | 53,58 |
| NaCl | - | 10 | 23,66 | 74,81 |
| Kontrolle | - | 10 | 0,0 | 0,0 |
| Gesamt | - | 65 | 54,3 | 137,93 |

Während Form II und die Kontrollen keinen beziehungsweise keinen relevanten Anstieg an TNF-alpha verursachen, kam es nach der Gabe von Form I zu deutlichen Erhöhungen im Vergleich zu den anderen Gruppen. Dieser Effekt war am deutlichsten in der 20 µg Dosierung zu sehen, diese Gruppe unterschied sich signifikant (p < 0,001) sowohl von den Kontrollgruppen als auch von den Gruppen die Form II erhielten. Hier tritt ein spezifisch immunstimulierender Effekt bei Form I auf, der bei Form II nicht zu beobachten ist.

### Beispiel 8 - Zytokine im Sepsismodell

In dem in Beispiel 3 beschriebenen Sepsismodell wurde auch untersucht welchen Einfluss die Verabreichung von Form I und Form II auf bestimmte Zytokine, insbesondere IL-6 und TNF-alpha, in mit S. pyogenes infizierten Mäusen zeigt. Die Blutentnahme erfolgte zu Versuchsende 48 Stunden nach der Infektion.

Da gezeigt werden konnte, dass die durch die Infektion verursachten Zytokinanstiege sich teilweise auch innerhalb der Kontrollgruppen (Negativkontrolle und NaCl) zwischen den Wiederholungen signifikant unterscheiden, war hier kein Zusammenführen der Daten aus den Wiederholungen möglich.

Einschränkend sei weiterhin erwähnt, dass auch diese Werte nur von jenen Tieren erfasst wurden, die auch den zweiten Versuchstag bis zum Ende überlebten. Die Erfinder vermuten außerdem, dass die vorher verendeten Tiere sehr hohe prognostisch ungünstige IL-6 Werte aufwiesen.

Deutliche Tendenzen einer IL-6-Reduktion durch Form II waren dennoch teilweise vorhanden, konnten jedoch leider nicht in allen Wiederholungen bestätigt werden. Weitere Tiermodelle, die besser geeignet sind die supprimierende Wirkung von Form II auf Zytokine auch in vivo eindeutig nachzuweisen werden derzeit von den Erfindern durchgeführt.

### Beispiel 9 - Leberenzyme/Transaminasen bei gesunden Versuchstieren

Bei der Verabreichung von Form I und Form II an gesunde Mäuse wurde untersucht, ob und welchen Einfluss die Applikation auf bestimmte Leberenzyme, insbesondere die Transaminasen GOT (AST) und GPT (ALT) zeigt. Die Blutentnahme erfolgte am Ende des zweiten Anwendungstages. Deutliche Unterschiede zwischen den Gruppen konnten hier nicht festgestellt werden. Dies bestätigt die in Beispiel 3 gezeigte gute Verträglichkeit.

### Beispiel 10 - Leberenzyme/Transaminasen im Sepsismodell

In dem in Beispiel 3 beschriebenen Sepsismodell wurde auch untersucht welchen Einfluss die Verabreichung von Form I und Form II bestimmte Leberenzyme, insbesondere die Transaminase GOT (AST) und GPT (ALT), in mit S. pyogenes infizierten Mäusen zeigt.

Die Blutentnahme erfolgte 48 Stunden nach der Infektion. Im Rahmen einer Infektion mit septischem Verlauf kann es zu teilweise drastischen Erhöhungen der Leberenzymwerte kommen. Analog zu den Zytokinwerten bei den infizierten Mäusen gab es auch hier signifikante Unterschiede innerhalb gleicher Gruppen zwischen den einzelnen Wiederholungen, so dass eine Gesamtauswertung der Daten nicht möglich war. Form II zeigte in manchen Wiederholungen Tendenzen die Leberenzymwerte zu senken. Diese Hinweise waren aber nicht in allen Wiederholungen erkenntlich.

Bedeutende Unterschiede zwischen den Gruppen konnten jedoch generell nicht gefunden werden. Analog zu den Keimzahlen in Beispiel 6 und den Zytokinwerten in Beispiel 8 liegt ein Bias durch die fehlenden Daten vorzeitig verendeter Tiere vor. Die Erfinder gehen davon aus, dass eine Bestimmung der Transaminasen nach 24 Stunden einen deutlichen Vorteil für die mit 5-Amino-2,3-dihydrophthalazin-1,4-dion Natriumsalz, insbesondere Form II behandelten Mäuse nachweisbar wäre.

### Herstellung der erfindungsgemäßen kristallinen Anhydratformen I und II

Nachfolgend wird die Herstellung der kristallinen Formen I und II beispielhaft beschrieben.

Syntheseausgang für alle Herstellungsbeispiele ist jeweils im Stand der Technik bekanntes Luminol, welches sich zum Beispiel nach folgendem Reaktionsschema herstellen lässt:

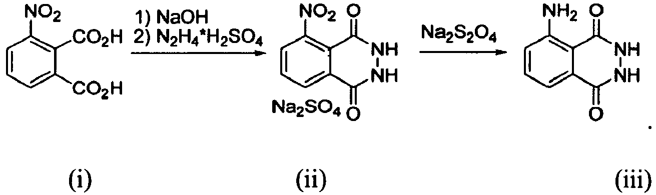

Dargestellt ist die Synthese von Luminol (iii) durch Umsetzung von 3-Nitrophthalsäure (i), das in alkalischem Medium durch Hydrazin oder einem seiner Salze, oder andere geeignete Reduktionsmittel, etwa Ammoniumsulfit oder Triethylenglykol, zu Luminol über 3-Nitrophthalanhydrid (ii) reduziert werden kann. Geeignete Herstellungsverfahren für Luminol finden sich schließlich in: Williamson, K. L. In: Macroscale and Microscale Organic Experiments; 2nd ed.; D.C. Heath: Lexington, MA, 1994. Ein weiteres für die Herstellung von Luminol geeignetes Verfahren, welches sich eines Raney-NickelKatalysators bedient, findet sich beispielsweise in der US 6,489,326 B1.

In einem spezielleren Verfahren kann das Ausgangsprodukt Luminol auch wie folgt hergestellt werden, wobei unter Verwendung der angegebenen Äquivalente auch beliebige Mengen Luminol hergestellt werden können:

### Herstellungsbeispiel Luminol

### 1. Reaktionsstufe: 3-Nitrophthalsäure - 3-Nitrophthalhydrazid Ansatzgrößen:

| Substanz | Äquivalent | Ansatz | Menge [mol] |
|---|---|---|---|
| 3-Nitrophtalsäure | 1 | 200 g | 0.95 |
| Hydrazinhydrat (98%) | 1.1 | 51 g (50 mL) | 1.02 |
| Ethylenglykol | 1.5 (vol/m) | 300 mL | N/A |
| Wasser | 6 (vol/m) | 1200 mL | N/A |
| Wasser zum Waschen | 3 x 1.5 (vol/m) | 900 mL (3 x 300mL) | N/A |

In einer ersten Stufe wurde 3-Nitrophthalsäure (200 g, 0.95 mol) und Hydrazinhydrat (51 g, 1.02 mol) vorgelegt und mit Ethylenglykol (300 mL) in einem 21 Reaktionskolben vermischt, der mit einer Kombination aus einem Rückflusskühler (T=110 °C) und einem Liebigkühler ausgestattet ist. Die Temperatur wurde bis auf 110 °C erhöht und das Wasser durch Destillation entfernt. Die Erwärmung der Reaktionsmischung wurde fortgeführt bis zum Rückfluss des Ethylenglykols bei ca. 200 °C. Nach einer Stunde bildete sich kein Wasser mehr. Die Mischung wurde auf ca. 100 °C abgekühlt und Wasser (1200 mL) hinzugefügt. Es entstand ein leicht bräunliches Präzipitat. Die Mischung wurde durch Zuführen von Eiswasser auf Raumtemperatur (25 °C ± 5 °C) abgekühlt und über Nacht gerührt. Das Präzipitat wurde filtriert und mit Wasser (3 x 300 mL) gewaschen. Das feuchte Produkt wurde getrocknet am Rotationsverdampfer bei 90 °C/20 ± 10 mbar bis zur Massenkonstanz.

Vorteile dieses Verfahrens liegen darin, dass durch die Verwendung von Hydrazinhydrat statt Hydrazinsulfat in den Folgeschritten kein störendes, anorganisches Material anfällt und dass durch die Verwendung von Ethylenglykol mit einem Siedepunkt von ca. 196 °C die Prozesskontrolle durch Rückflusskochen handhabbarer ist als bei anderen Lösemitteln mit höheren Siedepunkten, deren Verwendung zu Verunreinigungen im Produkt führen kann.

### 2. Reaktionsstufe: 3-Nitrophthalhydrazid - 5-Amino-2,3-dihydrophthalazin-1,4-dion Ansatzgrößen:

| Substanz | Äquivalent | Ansatz | Menge [mol] |
|---|---|---|---|
| 3-Nitrophtalhydrazid | 1 | 100 g | 0.48 |
| Natriumdithionit | 3.6 | 300 g | 1.73 |
| NaOH (3M) | 17 (vol/m) | 1700 mL | N/A |
| Essigsäure | 7.2 | 200 mL (210 g) | 3.5 |
| Wasser zum Waschen | 3 x ca. 1.5 (vol/m) | 510 mL (3x170 mL) | N/A |

In einer zweiten Stufe wird 3-Nitrophthalhydrazid zu 5-amino-2,3-dihydrophthalazin-1,4-dion umgesetzt, indem 3-Nitrophthalhydrazid (100 g, 0.48 mol) 48.3 mmol) in 3 molarer Natriumhydroxidlösung (1700 mL) gelöst wird unter Erwärmen auf ca. 50-60 °C. Zu dieser Lösung wird portionsweise das Natriumdithionit (300 g, 1.73 mol) eingetragen. Dabei steigt die Temperatur der Reaktionsmischung auf ca. 80 °C. Nach vollständiger Zugabe von Natriumdithionit wird die Reaktionsmischung für ca. 4 h zum Rückfluss erhitzt. Essigsäure (200 mL, 1,73 mol) wird zugegeben und die Reaktionsmischung über Nacht abgekühlt. Das resultierende Präzipitat wird isoliert und mit Wasser (3x170 mL) gewaschen. Das Produkt wird am Rotationsverdampfer bei ca. 80°C/20 ± 10 mbar getrocknet.

### Anhydratform I

### I. Herstellungsbeispiel I - kristalline Form I

Gegenstand der Erfindung ist ein Verfahren zur Herstellung der Anhydratform I durch Vermischen von 5-Amino-2,3-dihydrophthalazin-1,4-dion (Luminol) in Natronlauge und Eintropfen dieser Lösung in einen niedermolekularen flüssigen Alkohol, vorzugsweise Ethanol, der das Löslichkeitsprodukt des entstandenen Luminol Natriumsalzes so herabsenkt, dass letzteres auszufällen beginnt. Die Alkohole sollten einen Reinheitsgrad von vorzugsweise ≥ 95 %, besonders bevorzugt ≥ 98 %, aufweisen. Das durch Ausfällen entstandene Präzipitat wird erfindungsgemäß bei Temperaturen zwischen 50 und maximal 90°C getrocknet.

### Herstellunsgbeispiel I - kristalline Form I - Ausführungsform I

In einer bevorzugten Ausführungsform des Herstellungsbeispiels I lässt sich die kristalline Form I ausfällen durch Mischung von 500-750 mL einer 0,8 - 1,2 molaren Luminol Suspension mit von 500-750 mL einer 1,0-1,3 molaren Natronlösung, wobei die Mischung bei 20 - 50°C unter Rühren zu 10-15 l eines niedermolekularen Alkohols, bevorzugt Ethanol, bevorzugt mit einem Reinheitsgrad ≥ 95%, besonders bevorzugt ≥ 98 %, zugetropft wird, und die Suspension anschließend 15-25 h bei 10 - 40°C gerührt wird. Das entstandene Präzipitat wird nach Isolierung vorzugsweise an der Luft getrocknet. Nach weiterem Trocknen bei 50 °C - 80°C wird das Präzipitat in einer 10-20 fachen Menge niedermolekularen Alkohols, bevorzugt Ethanol mit einem Reinheitsgrad ≥ 95%, besonders bevorzugt ≥ 98 %, gelöst und die Suspension 15-25 h bei 10 -40°C gerührt und abfiltriert. Der Filterkuchen wird anschließend luftgetrocknet, bei 50°C- 90°C, bevorzugt 50°C, nachgetrocknet, pulverisiert und bis zur Erreichung eines Kristallwassergehalts von ≤ 0,4%, bevorzugt ≤ 0,3%, höchst bevorzugt ≤ 0,2%, getrocknet.

### Herstellunsgbeispiel I kristalline Form I - Ausführungsform II

a) In einer besonders bevorzugten Ausführungsform des Herstellungsbeispiels I lässt sich kristalline Form I wie folgt herstellen:
b) 190 - 220 g Luminol im 2 Becherglas vorlegen und unter Rühren bei mindestens 20°C in 1,25 L 1,0-1,1 -molarer NaOH-Lösung lösen.
c) Ansatzlösung über die Porzellannutsche mit Filterpapier 0 70 mm (z.B. Schleicher & Schuell Typ 1575) absaugen, ohne nachzuwaschen.
d) 12-14 l Ethanol, bevorzugt mit einem Reinheitsgrad ≥ 98%) im Dreihalsrundkolben vorlegen. Die Ansatzlösung unter Rühren bei 20 °C ± 10°C innerhalb 25 min ± 10 min zutropfen. Die Suspension 16 ± 5 h bei 20 °C ± 10 °C rühren lassen.
e) Das Präzipitat über eine Porzellannutsche mit Filterpapier absaugen und mit ca. 400 - 500 mL eines niedermolekularen Alkohols, bevorzugt Ethanol mit einem Reinheitsgrad ≥ 98%, nachwaschen.
f) Den Filterkuchen auf eine Glasschale fein verteilen und unter dem Abzug über Nacht trocknen. Anschließend im Trockenschrank bei 50°C- 90°C, bevorzugt 50°C-70°C, besonders bevorzugt 50°C, bis zur Massenkonstanz nachtrocknen.
g) Das Präzipitat pulverisieren und auswiegen.
h) Die 12 - 15 fache Menge (12-15 mL/g) des gewonnenen Präzipitats von g) an einem niedermolekularen Alkohol (z.B. Methanol, Ethanol, 2-Propanol, bevorzugt mit einem Reinheitsgrad ≥ 98 %) in einem 4 l Dreihalskolben vorlegen. Das Präzipitat unter Rühren darin suspendieren. Die Suspension 16 h ± 5 h bei 20 °C ± 10 °C rühren lassen. Die Suspension über eine Porzellannutsche mit Filterpapier absaugen und mit ungefähr 500 mL mLeines niedermolekularen Alkohols nachwaschen.
i) Den Filterkuchen auf einer Glasschale fein verteilen und unter dem Abzug über Nacht trocknen. Anschließend im Trockenschrank 2 - 6 h bei 50°C- 70°C, bevorzugt 50°C, bis zur Massenkonstanz trocknen. Die Substanz mörsern und auswiegen. Der Kristallwassergehalt soll ≤ 0,4 % sein, ermittelbar nach z.B. Karl-Fischer-Titrationsmethode. Ist der Kristallwassergehalt > 0,4 %, Schritte unter h) - i) wiederholen.
j) Substanz auswiegen und Ausbeute ermitteln.

### Herstellungsbeispiel I kristalline Form I -Ausführungsform III

In einer höchst bevorzugten Ausführungsform lässt sich kristalline Form I wie folgt herstellen:
a) 200 g Luminol im 21 Becherglas vorlegen und unter Rühren bei 30 °C ± 10 °C in 1,25 Liter 1-molarer NaOH-Lösung lösen.
b) Ansatzlösung über die Porzellannutsche mit Filterpapier Ø 70 mm (z.B. Schleicher & Schuell Typ 1575) absaugen, ohne nachzuwaschen.
c) 12,5 l Ethanol, bevorzugt mit einem Reinheitsgrad ≥ 99%, im 20 l Dreihalsrundkolben vorlegen. Die Ansatzlösung unter Rühren bei 25 °C ± 5 °C innerhalb 30 min ± 5 min zutropfen. Die Suspension 20 h ± 1 h bei 25 °C ± 5 °C rühren lassen.
d) Die Suspension über eine Porzellannutsche mit Filterpapier Ø 185 mm (z.B. Schleicher & Schuell Typ 1575) absaugen und mit ca. 500 mL Ethanol, bevorzugt mit einem Reinheitsgrad ≥ 99 %, nachwaschen. Den Filterkuchen auf eine Glasschale fein verteilen und unter dem Abzug trocknen. Anschließend im Trockenschrank bei 50°C -70°C, bevorzugt 50°C, bis zur Massenkonstanz trocken.
e) Das Präzipitat pulverisieren und auswiegen.
f) Die 12-fache Menge (12 mL/g) des gewonnenen Präzipitats an Ethanol, bevorzugt mit einem Reinheitsgrad ≥ 99 %, im Dreihalskolben vorlegen. Die Substanz unter Rühren darin suspendieren. Die Suspension 20 h ± 1 h bei 25 °C ± 5 °C rühren lassen. Die Suspension über eine Porzellannutsche mit Filterpapier Ø 185 mm (z.B. Schleicher & Schuell Typ 1575) absaugen und mit ca. 500 mL Ethanol mit einem Reinheitsgrad von ≥ 95 %, bevorzugt ≥ 98 %, nachwaschen. Den Filterkuchen auf einer Glasschale fein verteilen und unter dem Abzug trocknen. Anschließend im Trockenschrank bei 50 °C - 70°C, bevorzugt 50°C bis zur Massenkonstanz trocknen. Das Produkt mörsern und auswiegen.
g) Der Kristallwassergehalt soll ≤ 0,4 % sein, ermittelbar z.B. mit Karl-Fischer-Titration. Ist der Kristallwassergehalt > 0,4 %, Schritte unter f)- g) wiederholen.
h) Substanz auswiegen und Ausbeute ermitteln.

### Herstellungsbeispiel II kristalline Form I

Unter wässrigen Bedingungen lässt sich, ausgehend von Luminol, kristalline Form I dadurch herstellen, dass eine Natriumhydroxidlösung hergestellt wird, in die Luminol eingetragen wird. Das Luminol wird durch Rühren aufgelöst. Anschließend wird Ethanol innerhalb von 10-40 min zugeführt, wobei das Luminol als Salz ausfällt. Nach vollständiger Zugabe und Rühren über mehrere Stunden wird die Suspension gefiltert, der Filterkuchen gewaschen und getrocknet.

### Herstellungsbeispiel II Form I - Ausführungsform II

In einer bevorzugten Ausführungsform wird kristalline Form I unter Verwendung folgender Äquivalente an Reaktanden hergestellt: Es wird eine Lösung aus 1.0-1.4 Äquivalenten Natriumhydroxid in 4-7 vol/m Wasser hergestellt, in die 1 Äquivalent Luminol eingetragen wird. Die Reaktionsmischung wird bis zur vollständigen Lösung gerührt. Anschließend wird bei Raumtemperatur (25 °C ± 5°C) innerhalb von ca. 10-40 min Ethanol (50-70 vol/m) zugetropft. Dabei fällt das Luminol Natriumsalz als Niederschlag aus.

Nach vollständiger Zugabe wird die Reaktionsmischung über mehrere Stunden bei Raumtemperatur (25 °C ± 5 °C) nachgerührt und die Suspension gefiltert; der Filterkuchen wird mit Ethanol (ca. 10-15 vol/m) gewaschen und wahlweise im Vakuum-Trockenschrank oder an einem Rotationsverdampfer getrocknet.

### Herstellungsbeispiel II - Form I - Ausführungsform III

In einer besonders bevorzugten Ausführungsform wird die kristalline Form I unter Verwendung der folgenden Äquivalente an Reaktanden hergestellt:

| Substanz | Äquivalente | Ansatz | Menge [mol] |
|---|---|---|---|
| Luminol | 1 | 82 g | 0.46 |
| Natriumhydroxid | 1.2 | 22 g | 0.55 |
| Wasser für Luminol Lösung | 6 (vol/m) | 490 mL | N/A |
| Ethanol abs. | 60 (vol/m) | 4.900 mL | N/A |
| Ethanol abs. zum Waschen | ca. 13 (vol/m) | 1.050 mL (3x350 mL) | N/A |

Es wird eine Lösung aus 1.0 -1.4 Äquivalent Natriumhydroxid, bevorzugt 1.2 Äquivalent Natriumhydroxid (22 g, 0,55 mol), in Wasser (6 vol/m) hergestellt (101 Reaktor).

In die Natriumhydroxid-Lösung wird 1 Äquivalent Luminol eingetragen. Die Reaktionsmischung wird bis zur vollständigen Lösung gerührt. Es resultiert eine braune klare Lösung.

Anschließend wird bei Raumtemperatur (25 °C ± 5°C) innerhalb von ca. 20 min. Ethanol (60 vol/m) zugetropft. Dabei fällt das Luminol Natriumsalz als Niederschlag aus.

Nach vollständiger Zugabe wird die Reaktionsmischung für maximal 20 h, bevorzugt 2 -8 h, besonders bevorzugt 8 h bei Raumtemperatur (25 °C ± 5 °C) nachgerührt und die Suspension gefiltert; der Filterkuchen wird mit Ethanol (ca. 13 vol/m) gewaschen und wahlweise im Vakuum-Trockenschrank bei 50-90 °C /1-3 mbar bevorzugt 50-70°C, besonders bevorzugt 50°C, oder an einem Rotationsverdampfer bei 20 ± 10 mbar und 50°C-90 °C, bevorzugt 50°-70°C, besonders bevorzugt 50°C, getrocknet.

### Herstellunsgbeispiel III Form I - skalierbare Ansatzgrößen

Die Erfinder haben gefunden, dass sich diese aufgefundenen Äquivalentrelationen für beliebige Ansatzgrößen an Luminol eignen und reproduzierbar die Herstellung der gewünschten Form I erlauben.

### Anhydratform II:

### Herstellungsbeispiel I für kristalline Form II

Die Erfinder haben ein Verfahren zur Herstellung kristalliner Form II gefunden, bei dem unter wässrigen Bedingungen Luminol mit einer Natriumhydroxidlösung vermischt und durch Hinzufügen von 2-Propanol das Löslichkeitsprodukt des Luminol Natriumsalzes derart herabgesetzt wird, dass letzteres auszufällen beginnt. Das ausgefällte Luminol Natriumsalz wird mit 2-Propanol gewaschen und bis zur Massenkonstanz getrocknet.

### Herstellungsbeispiel I kristalline Form II - Ausführungsform II

In einer bevorzugten Ausführungsform wird kristalline Form II unter Verwendung folgender Äquivalente an Reaktanden hergestellt. Es wird eine Lösung aus 1.0 - 2.0 Äquivalenten Natriumhydroxid, vorzugsweise 1.1- 1.4 Äquivalente Natriumhydroxid, besonders bevorzugt 1.2 Äquivalenten Natriumhydroxid, in 6-7.5 vol/m Wasser hergestellt, in die 0.5-1 Äquivalente Luminol eingetragen wird. Die Reaktionsmischung wird bis zur vollständigen Lösung gerührt. Anschließend wird bei Raumtemperatur (25 °C ± 5°C) innerhalb von ca. 10-40 min 2-Propanol (60-120 vol/m) zugetropft. Dabei fällt das Luminol Natriumsalz als Niederschlag aus. Nach vollständiger Zugabe wird die Reaktionsmischung bei Raumtemperatur (25 °C ± 5 °C) gerührt. Das Produkt wird gefiltert, mit einem niedermolekularen Alkohol, vorzugsweise 2-Propanol, gewaschen (ca. 13-16 vol/m) und wahlweise im Vakuum-Trockenschrank bei 85 °C-120°C /1-3 mbar, vorzugsweise 90 °C/1-3 mbar, oder am Rotationsverdampfer bei 85 °C-120°C/20 ± 10 mbar, getrocknet.

### Herstellungsbeispiel I kristalline Form II - Ausführungsform III

In einer besonders bevorzugten Ausführungsform werden Äquivalente und Ansatzgrößen für die Herstellung kristalliner Form II dargestellt, wobei dies für beliebige Ansatzgrößen an Luminol gilt und welche im Nachfolgenden beispielhaft für eine Ansatzgröße an Luminol von 10 g dargestellt ist:

| Substanz | Äquivalente | Ansatz | Menge [mmol] |
|---|---|---|---|
| Luminol | 1 | 10 g | 56.5 |
| Natriumhydroxid | 1,2 | 2.7 g | 67.5 |
| Wasser für Luminol Lösung | 6 (vol/m) | 60 mL | N/A |
| 2-Propanol (abs.) | 60 (vol/m) | 600 mL | N/A |
| 2-Propanol (abs.) zum Waschen | 15 (vol/m) | 150 mL (3x50 mL) | N/A |

Es wird 1.2 Äquivalent an Natriumhydroxid und 1 Äquivalent an Luminol in Wasser (6 vol/m) aufgelöst. Es bildet sich eine klare Lösung. Diese Lösung sollte sofort weiterverarbeitet werden, da sie nachdunkelt. Anschließend wird bei Raumtemperatur (25 °C ± 5° C) innerhalb von ungefähr 20 min 2-Propanol (60 vol/m) hinzugefügt, wobei sich ein Niederschlag von Luminol Natriumsalz bildet. Die Suspension wird gerührt bei Raumtemperatur für 1 - 5 h, bevorzugt 2 h, besonders bevorzugt 3 h. Das Produkt wird gefiltert, mit 2-Propanol (ca. 15 vol/m), gewaschen und wahlweise im Vakuum-Trockenschrank getrocknet bei 85 °C-120°C /1-3 mbar, bevorzugt 90 °C/1-3 mbar oder am Rotationsverdampfer bei 85 °C-120°C/20 ± 10 mbar, bevorzugt 90 °C/ 20 ± 10 mbar bis zur Massenkonstanz.

### Herstellungsbeispiel I für kristalline Form II - Ausführungsform IV

Die Erfinder zeigen ferner ein Verfahren auf, das sich für Ansatzgrößen an Luminol von mindestens 300 g, bevorzugt 400 g, besonders bevorzugt ≥ 500 g, eignet und beispielhaft für eine Ansatzgröße von 785 g aufgezeigt wird:

| Substanz | Äquivalente | Ansatz | Menge [mol] |
|---|---|---|---|
| Luminol | 1 | 785 g | 4.43 |
| Natriumhydroxid | 1.2 | 212 g | 5.32 |
| Wasser für Luminol Lösung | 6 (vol/m) | 4700 mL | N/A |
| 2-Propanol abs. | 60 (vol/m) | 47000 mL | N/A |
| 2-Propanol abs. zum Waschen | 13 (vol/m) | 10.51 (3x3.5 l) | N/A |

Es wird eine Lösung aus 212 g (5.32 mol, 1.2 eq.) Natriumhydroxid in 4.700 mL Wasser hergestellt. (80 l Reaktor).Das Luminol (785 g, 4.43 mol) wird in die Natriumhydroxid Lösung eingetragen und bis zu seiner Auflösung gerührt. Es resultiert eine klare, braune Lösung, in die 2-Propanol (60 vol/m) über einen Zeitraum von 20-30 min, bevorzugt 30 min, zugepumpt wird. Dabei fällt das Luminol Natriumsalz als Niederschlag aus. Nach vollständiger Zugabe wird die Mischung für mindestens 10 h, bevorzugt 12 h, bei Raumtemperatur (25 °C ± 5 °C) nachgerührt. Die Mischung wird filtriert, der Filterkuchen mit 2-Propanol (13 vol/m) gewaschen und wahlweise im Vakuum-Trockenschrank bei 85 °C-120°C /1-3 mbar, bevorzugt 90 °C/1-3 mbar oder am Rotationsverdampfer bei 85 °C-120°C/20 ± 10 mbar, bevorzugt 90 °C/ 20 ± 10 mbar bis zur Massenkonstanz getrocknet.

### Herstellungsbeispiel I -kristalline Form II - skalierbare Ansatzgrößen

Die Erfinder haben gefunden, dass sich diese aufgefundenen Äquivalentrelationen für beliebige Ansatzgrößen an Luminol eignen und reproduzierbar die Herstellung der gewünschten Form II erlauben. Bei der Verwendung von größeren Mengen an Luminol (≥ 500 g) und Volumina ist darauf zu achten, dass die Rührdauer entsprechend lang gewählt wird, um eine möglichst hohe Ausbeute an Endprodukt zu erhalten.

### Herstellungsbeispiel II für kristalline Form II (Umkristallisation)

Die Erfinder haben gefunden, dass sich kristalline Form II durch Umkristallisation aus kristalliner Form I unter Verwendung folgender Äquivalente herstellen lässt:
In eine Lösung aus 1 Äquivalent Form I wird 2-Propanol (10 vol/m) mit einem Reinheitsgrad von 70-90%, bevorzugt 80-90%, besonders bevorzugt 90%, eingetragen und mindestens 10-14 h, bevorzugt 10-12, bei Raumtemperatur (25 °C ± 5 °C) gerührt. Die Mischung wird filtriert und der Filterkuchen mit 2-Propanol (ca. 20 vol/m) gewaschen und wahlweise im Vakuum-Trockenschrank bei 85°C-120°C /1-3mbar, bevorzugt 90°C/1-3mbar, oder am Rotationsverdampfer bei 85°C-120°C /20 mbar ± 10 mbar bis zur Massenkonstanz getrocknet.

### Herstellungsbeispiel II für kristalline Form II - Umkristallisation aus Form I - skalierbare Ansatzgrößen

Ein von den Erfindern gefundenes Umkristallisationsverfahren lässt sich unter Verwendung der folgenden Äquivalente für beliebige Ansatzgrößen verwenden, das beispielhaft für eine Ansatzgröße an Form I von 1 g beschrieben wird:

| Substanz | Äquivalente | Ansatz | Menge [mmol] |
|---|---|---|---|
| Form II | 1 | 1 g | 5,02 |
| Wasser für Lösung 2-Propanol | 1 (vol/m) | 1 mL | N/A |
| 2-Propanol | 9 (vol/m) | 9 mL | N/A |
| 2-Propanol zum Waschen | ca. 20 (vol/m) | 20 mL (2 x 10 mL) | N/A |

Kristalline Form I (1 g) wird in wässrigem 2-Propanol (10-20 % Wasser) suspendiert und mindestens 10 h, bevorzugt 10-14 h, besonders bevorzugt 10-12 h gerührt bei Raumtemperatur (25 °C ± 5 °C) gerührt. Nach Filtration des verbliebenen Lösungsmittels wird der Filterkuchen mit 2-Propanol gewaschen (2x 10 mL) und am Rotationsverdampfer bei 90 °C/20 mbar bis Massenkonstanz getrocknet.

**Abkürzungsverzeichnis:**

| | |
|---|---|
| µg | Mikrogramm |
| aaO. | am angegebenen Orte |
| ALT | Alanin-Aminotransferase |
| AST | Aspartat-Aminotransferase |
| ca. | circa |
| DMSG | Deutsche Multiple Sklerose Gesellschaft |
| EMEA | European Medicines Agency |
| g | Gramm |
| GOT | Glutamat-Oxalacetat-Transaminase |
| GPT | Glutamat-Pyruvat-Transaminase |
| IL | Interleukin |
| KBE | Koloniebildende Einheiten |
| l | Liter |
| LPS | Lipopolysaccharid |
| µg | Mikrogramm |
| mL | Milliliter |
| MW | Mittelwert |
| ng | Nanogramm |
| pg | Pikogramm |
| REM | Rasterelektronenmikroskop |
| STD | Standardabweichung |
| TNF | Tumor Nekrose-Faktor |
| U | Internationale Einheiten |
| vol/m | Volumeneinheit pro Masseeinheit |
| Z.B. | Zum Beispiel |

## Patentansprüche

1. Kristallformen I und II des 5-Amino-2,3-dihydrophthalazin-1,4-dion Natriumsalzes zur Behandlung von entzündlichen Erkrankungen, **gekennzeichnet durch** mittels Röntgenpulverdiagrammen ermittelten Kristallographie-Werte:
d-Werte: 13,5; 6,9; 5,2; 4,6; 3,9; 3,5; 3,4; 3,3; 3,1; 3,0 und
2-Theta-Werte: 6,5; 12,7; 16,9; 19,3; 22,8; 25,8; 26,6; 27,2; 28,7; 30,3 für Form I
sowie
d-Werte: 12,9; 7,9; 7,1; 6,5; 5,3; 4,0; 3,7; 3,6; 3,3; 3,2 und
2-Theta-Werte: 6,8; 11,2; 12,5; 13,7; 16,7; 22,4; 24,3; 24,9; 27,2; 27,8 für Form II.

2. Kristallformen I und II zur Behandlung von entzündlichen Erkrankungen gemäß Anspruch 1, **gekennzeichnet dadurch, dass** es sich bei den entzündlichen Erkrankungen um Zustände mit überschießender Immunreaktion handelt.

3. Kristallformen I und II zur Behandlung von Zuständen mit überschießender Immunreaktion gemäß Anspruch 2, **dadurch gekennzeichnet, dass** es die überschießenden Immunreaktionen bei Abstoßungsreaktionen nach Transplantationen, aktiven Autoimmunerkrankungen, aplastischer Anämie, Pemphigus, Pemphigoid, endogener Uveitis, nephrotischem Syndrom, atopischer Dermatitis oder septischen Zuständen auftreten.

4. Kristallformen I und II zur Behandlung von aktiven Autoimmunerkrankungen gemäß Anspruch 3, **dadurch gekennzeichnet, dass** es sich bei den aktiven Autoimmunerkrankungen um aktive Rheumatoide Arthritis, schubförmige Multiple Sklerose, lupoide Hepatitis, Polyarteriitis nodosa, Morbus Crohn, Colitis ulcerosa, Dermatomyositis. Morbus Behcet, Morbus Behcet Uveitis, thrombozytopenische Purpura, Myasthenia gravis, Polymyositis, Psoriasis, Psoriasis Arthritis, Morbus Bechterew, paroxysmale nächtliche Hämoglobinurie, ankylosierende Spondylitis oder Autoimmun-Thyreoiditis handelt.

5. Kristallformen I und II zur Behandlung von septischen Zuständen gemäß Anspruch 3, **dadurch gekennzeichnet, dass** es sich bei den septischen Zuständen um bakterielle Infektionen oder ein durch immunologische oder chemische Faktoren ausgelöstes Systemisches Inflammatorisches Response-Syndrom (SIRS) handelt.

6. Kristallformen I und II zur Behandlung von septischen Zuständen ausgelöst durch bakterielle Infektionen gemäß Anspruch 5, **dadurch gekennzeichnet, dass** es dabei um Infektionen mit Methicillin-resistentem *Staphylokokkus aureus* (MRSA) handelt.

7. Kristallformen I und II zur Behandlung von entzündlichen Erkrankungen gemäß Anspruch 1, **gekennzeichnet dadurch, dass** es sich bei den entzündlichen Erkrankungen um Zustände mit immundefizientem Hintergrund handelt.

8. Kristallformen I und II zur Behandlung von Zuständen mit immundefizientem Hintergrund gemäß Anspruch 7, **dadurch gekennzeichnet, dass** es sich bei den Zuständen mit immundefizientem Hintergrund um grippale Infekte, rezidivierende Atemwegsinfekte, rezidivierende Infekte der ableitenden Harnwege, Müdigkeit, Schwäche, Konzentrationsstörungen unbekannter Genese, Rekonvaleszenz, chronische Virusinfektionen, onkologische Erkrankungen, septische Granulomatose, Neutropenie, Feigwarzen, Keratosen, Autoimmunerkrankungen, Radiogene Kolitis, Divertikelkrankheit, Allergien, Enteritis oder Kolitis handelt.

9. Kristallformen I und II zur Behandlung von chronischen Virusinfektionen gemäß Anspruch 8, **dadurch gekennzeichnet, dass** es sich bei den Virusinfektionen um HIV, Hepatitis B, Hepatitis C, Enzephalitis, Herpes zoster, Herpes simplex, Innenohrinfekte, Varizellen, Masern, Zytomegalie oder Epstein-Barr-Virus handelt.

10. Kristallformen I und II zur Behandlung von onkologischen Erkrankungen gemäß Anspruch 8, **dadurch gekennzeichnet, dass** es sich bei den onkologischen Erkrankungen um Haarzellenleukämie, Myeloische Leukämie, Multiples Myelom, Follikuläre Lymphome, Kaposi-Sarkom, Kutanes T-Zell-Lymphom, Nasopharynxkarzinom, Karzinoid, Nierenkarzinom, Harnblasenkarzinom, Basalzellkarzinome, metastasierende Karzinome oder Malignes Melanom handelt.

11. Kristallformen I und II zur Behandlung von Autoimmunerkrankungen gemäß Anspruch 8, **dadurch gekennzeichnet, dass** es sich bei der Autoimmunerkrankung um schubförmige Multiple Sklerose handelt.

12. Kristallformen I und II zur Behandlung von Allergien gemäß Anspruch 8, **dadurch gekennzeichnet, dass** es sich bei den Allergien um Heuschnupfen, Polymorphe Lichtdermatose, Ekzeme oder Neurodermitis handelt.

13. Kristallformen I und II zur begleitenden Behandlung von Zuständen mit immundefizientem Hintergrund gemäß Anspruch 8 während und nach Chemo- oder Strahlentherapie.

14. Kristallformen I und II zur Behandlung von chronischen entzündlichen Erkrankungen gemäß Anspruch 1, **gekennzeichnet durch** einen wechselnden Immunstatus der chronisch entzündlichen Erkrankung.

15. Kristallformen I und II zur Behandlung von chronischen entzündlichen Erkrankungen mit wechselndem Immunstatus gemäß Anspruch 14, **dadurch gekennzeichnet, dass** es sich bei den chronisch entzündlichen Erkrankungen mit wechselndem Immunstatus um Multiple Sklerose, Hepatitis C, chronische Enteritiden oder Kolitiden handelt.
